# EUROPEAN PATENT SPECIFICATION

(11) **EP 1 388 013 B1**
(45) Date of publication and mention of the grant of the patent: **11.07.2007**
(21) Application number: 02713418.8
(22) Date of filing: 15.01.2002
(51) Int. Cl.: G01N 33/574, A61K 49/00, A61K 51/00

(54) **COMPOSITIONS AND METHODS FOR THE USE OF FIBRONECTIN FRAGMENTS IN THE DIAGNOSIS OF CANCER**
ZUSAMMENSETZUNGEN UND VERFAHREN ZUR VERWENDUNG VON FIBRONEKTIN-FRAGMENTEN BEI KREBSDIAGNOSE
COMPOSITIONS ET METHODES DESTINEES A L'UTILISATION DE FRAGMENTS DE FIBRONECTINE DANS LE DIAGNOSTIC DU CANCER

(30) Priority: 18.01.2001 US 765496
(43) Date of publication of application: 11.02.2004
(73) Proprietor: THE REGENTS OF THE UNIVERSITY OF MICHIGAN, Ann Arbor, Mi 48109-1280 (US)
(72) Inventor: LIVANT, Donna, Ann Arbor, MI 48105 (US)
(74) Representative: Müller Fottner Steinecke
(86) International application number: PCT/US2002/001189
(87) International publication number: WO 2002/057786

(56) References cited:
- US-A- 4 894 326
- US-A- 5 840 514
- PELTONEN J ET AL: "FIBRONECTIN GENE EXPRESSION BY EPITHELIAL TUMOR CELLS IN BASAL CELLCARCINOMA: AN IMMUNOCYTOCHEMICAL AND IN SITU HYBRIDIZATION STUDY" JOURNAL OF INVESTIGATIVE DERMATOLOGY, NEW YORK, NY, US, vol. 91, no. 4, October 1988 (1988-10), pages 289-293, XP000984049 ISSN: 0022-202X
- LIVANT DONNA L ET AL: "Invasion of selectively permeable sea urchin embryo basement membranes by metastatic tumor cells, but not by their normal counterparts." CANCER RESEARCH, vol. 55, no. 21, 1995, pages 5085-5093, XP001147064 ISSN: 0008-5472
- ALBRECHT M ET AL: "Fibronectin in human prostatic cells in vivo and in vitro: Expression, distribution, and pathological significance" HISTOCHEMISTRY AND CELL BIOLOGY, SPRINGER, BERLIN, DE, vol. 112, no. 1, July 1999 (1999-07), pages 51-61, XP002218305 ISSN: 0948-6143
- LIVANT D L ET AL: "THE PHSRN SEQUENCE INDUCES EXTRACELLULAR MATRIX INVASION AND ACCELERATES WOUND HEALING IN OBESE DIABETIC MICE" JOURNAL OF CLINICAL INVESTIGATION, NEW YORK, NY, US, vol. 105, no. 11, June 2000 (2000-06), pages 1537-1545, XP001147066 ISSN: 0021-9738
- LIVANT D L ET AL: "ANTI-INVASIVE, ANTITUMORIGENIC, AND ANTIMETASTATIC ACTIVITIES OF THE PHSCN SEQUENCE IN PROSTATE CARCINOMA" CANCER RESEARCH, AMERICAN ASSOCIATION FOR CANCER RESEARCH, BALTIMORE, MD, US, vol. 60, no. 2, 15 January 2000 (2000-01-15), pages 309-320, XP001147065 ISSN: 0008-5472

## Description

### FIELD OF THE INVENTION

The present invention relates to the detection and imaging of cancer *in vivo* and *in vitro.*

### BACKGROUND

Early detection of metastatic solid tumors allows for timely therapeutic interventions which result in a greater incidence of tumor remission and improved long term survival as compared tumor detection at a more advanced stage of a metastatic disease. Traditional diagnostic methods, however, often rely on the palpation of a tumor mass. For example, on physical examination, detection of a tumor in the breast, prostate, or cervix requires a tumor mass large enough to be palpated by human touch. Similarly, standard x-ray protocols only image a tumor once it has divided to a mass sufficient to differentially absorb or transmit (as compared to normal tissue) gamma energy, thereby, producing a spot or shadow on an exposed photosentive emulsion.

By the time many solid tumors have grown to a mass sufficient to be detected, according to the methods outlined above, they are less susceptible to therapeutic interventions and / or have metastasized to other sites in the body. This pre-diagnostic tumor growth increases the mortality and morbidity, associated with a given cancer, regardless of any post-diagnostic therapeutic intervention.

What is needed, therefore, is a method to detect a metastatic tumor (in a variety of tissues) before it progresses to a stage where it is resistant to therapeutic intervention(s). Importantly, this detection method may be used in concert with imaging techniques designed to reveal the anatomical *situs* of an early metastatic growth.

### SUMMARY OF THE INVENTION

The present invention relates to the detection of tumors *in vivo,* the imaging of tumors *in vivo,* and the imaging of cancerous tissue in pathological samples. In addition, the present invention provides compounds and methods useful in the detection of inflammatory and autoimmune pathologies including, but not limited to psoriasis, retinopathy, and arthritis.

In one embodiment, the present invention provides: A) an assay for detection of tumor cells *in vivo;* B) methods and materials associated with the imaging of tumor cells *in vivo;* and C) methods and materials for the detection of cancerous tissues in pathological samples. A variety of formats are contemplated for tumor cell detection assays. In one embodiment, an aliquot from a fluid sample obtained from a patient (being screened for the presence of a solid tumor) is contacted with a tissue culture comprising normal microvascular cells growing on the surface of a fibronectin-free substrate. Thereafter, the response of normal microvascular cells to the patient fluid sample is assessed. Where the patient fluid sample induces invasion of the normal microvascular cells into the fibronectin-free substrate, the patient can be considered at risk for having a metastatic growth *in vivo.* Where there is no significant invasion of the normal microvascular cells into the fibronectin-free substrate, the patient can be considered (at that time) free from metastatic growth.

In one embodiment, the present invention contemplates a method of detecting cancer *in vivo* comprising: a) providing: i) a human patient and ii) normal microvascular cells growing on a fibronectin-free substrate; b) obtaining a fluid sample from said patient; c) contacting said normal microvascular cells growing on a fibronectin-free substrate with said fluid sample; and d) detecting the invasion of said normal microvascular cells into said substrate. Preferably the normal microvascular cells are cultured (or at least briefly maintained) in serum-free culture media so as to essentially avoid introducing complicating serum factors.

While it is not intended that the present invention be limited to any specific mechanism, it is widely believed that tumorigenesis requires invasion by normal microvascular cells, which form the blood vessels that allow the circulation to oxygenate and nourish the tumor as it grows. Thus, the ability to stimulate microvascular cell invasion, which gives rise to angiogenesis, is important for tumor growth. [Wilson, C.L. et al., Matrilysin: an epithelial matrix metalloproteinase with potentially novel function. Int. J. Biochem. & Cell Biol. 28: 123-136 (1996), and Fingleton, B.M., et al. Matrilysin in early stage intestinal tumorigenesis. APMIS 107: 102-110 (1999).]

Matrix metalloproteinase (MMP) 7 is an example of an enzyme expressed early in epithelial tumorigenesis, which is known to cleave fibronectin to generate fragments that could stimulate invasion. Expression of this enzyme by tumor cells has been shown to stimulate endothelial cell invasion of the growing tumor, thus permitting the neovascularization that supports tumor growth. The fragmentation of plasma fibronectin (pFn) may cause endothelial cell invasion of the tumor as fragments of the pFn cell-binding domain are known to interact with the α5β1 integrin fibronectin receptors of microvascular cells to stimulate invasion. [Mogford, J. E., Davis, G.E., and Neininger, G.A., RGDN peptide interaction with endothelial α5β1 integrin causes sustained endothelin-dependent vasoconstriction of rat skeletal muscle arterioles. J. Clin. Invest. 100: 1647-1653. (1997).]

By extension, normal microvascular cells exposed to a fluid sample from a patient (having a metastatic growth) are potentially rendered capable of invading the substrate. Again, while it is not intended that the present invention be limited to any specific mechanism, enzymes expressed by a growing tumor may cleave fibronectin into fragments, containing the PHSRN sequence, which binds to the α5β1 receptor on the normal microvascular cell and thereby induces invasion of the substrate. More specifically, these PHSRN containing fibronectin fragments correspond to protein with an observed molecular weight of approximately 39kDa.

In one embodiment the present invention contemplates a method of detecting a metastatic growth *in vivo* comprising: a) providing: a human patient; b) obtaining a fluid sample from said patient; c) resolving the proteins within said fluid sample by gel electrophoresis; d) cross reacting said resolved proteins with any antibody recognizing fibronectin; e) detecting any interaction between said antibody and a protein with an observed molecular weight of approximately 39kDa. In a preferred embodiment, said antibody which reacts against fibronectin is an antibody that specifically binds to the PHSRN sequence. In one embodiment said fibronectin recognizing antibody is linked to a detectable marker. In a preferred embodiment, said antibody, specific for the PHSRN sequence, is linked to a detectable marker. In one example said detectable marker is a radioisotope. In another example said detectable marker is a fluorescent compound.

Where an interaction is detected between a resolved protein with an observed molecular weight of approximately 39kDa and an antibody which binds a peptide comprising the PHSRN sequence, the patient is considered at risk for having a cancerous growth. If substantially no interaction is detected between a resolved protein with an observed molecular weight of approximately 39kDa and an antibody whose epitope consists of the PHSRN sequence the patient is considered free (at that time) of metastatic growth.

It is also contemplated the present invention may be used as a cancer screening method for specific population of people at a higher risk for cancer as compared to the overall population. It is not intended that the present invention be limited to a specific risk factor. Exemplary risk factors include women with known genetic markers for breast cancer (i.e. braca), men and women with a familial history of colon cancer, and men with a familial history for prostate cancer

In one embodiment the present invention contemplates a method of detecting cancer comprising providing: i) a fluid sample from a subject and ii) cells disposed on a fibronectin-depleted substrate, wherein said cells are disposed under conditions such that said cells do not invade said substrate; contacting cells with said fluid sample; and measuring the extent of invasion of said cells into said substrate, wherein the detection of invasion indicates the presence of cancer in said subject.

In one embodiment said cells are microvascular cells. In another embodiment these microvascular cells are selected from the group of epithelial cells, fibroblasts, and keritinocytes. The present invention also contemplates an embodiment wherein prior to contacting said cell with said fluid, said cells are cultured in serum-free media. In a preferred embodiment, said fluid sample is selected from the group consisting of whole blood, plasma, and serum.

The present invention also contemplates an embodiment, wherein said fibronectin-depleted substrate is such that said normal cells are not capable of invading said substrate the without exposure to a fluid sample comprising the peptide comprising PHSRN.

The present invention also contemplates a composition comprising a peptide wherein said peptide is characterized by: a) an apparent molecular weight of 35 to 49 kDa as determined by 10% sodium dodecyl sulfate polyacrylamide under reducing conditions; and b) the ability to induce cells to invade a fibronectin free substrate. In a preferred embodiment this peptide has an apparent molecular weight of 39 kDa.

The present invention also contemplates a method of detecting cancer comprising, a) providing: i) a fluid sample taken from a subject; b) resolving the proteins in said fluid sample by gel electrophoresis; c) contacting said resolved proteins with an antibody that reacts against the epitope comprising PHSRN; and d) measuring the binding of antibody with resolved proteins having an apparent molecular weight of 35 to 49 kDa.

### DESCRIPTION OF THE FIGURES

Figure 1 schematically shows the one embodiment of the substrate used according to the present invention for testing tumor cells. The spatial relationship of the ectoderm of the *Strongylocentrotus purpuratus* embryo to its extracellular matrix and to blastocoelar structures are shown (s, spicules; h, hyalin layer; e, ectoderm; b, subectodermal basement membrane; bl, blastocoel; g, stomach of the primitive gut; c, coelomic pouches). The esophagus and intestine do not appear on the side of the embryo shown.
Figure 2 is a graph showing the results of the testing of normal cells on fibronectin-depleted substrates *in vitro* with and without invasion-inducing agents according one embodiment of the method of the present invention.
Figure 3A is a graph showing the results of inhibiting serum-induced human breast cancer cell invasion of the SU-ECM substrate with varying concentrations of the PHSCN peptide.
Figure 3B is a graph showing the results of inhibiting PHSRN-induced invasion by both human breast cancer cells and normal human mammary epithelial cells of the SU-ECM substrate with varying concentrations of the PHSCN peptide.
Figure 4A is a graph showing the results of inhibiting serum-induced human prostate cancer cell invasion of the SU-ECM substrate with varying concentrations of the PHSCN peptide.
Figure 4B is a graph showing the results of inhibiting PHSRN-induced invasion by both human prostate cancer cells and normal prostate epithelial cells of the SU-ECM substrate with varying concentrations of the PHSCN peptide.
Figure 5 is a graph showing the results of inhibiting serum-induced rat prostate cancer cell invasion of the SU-ECM substrate with varying concentrations of the PHS(homo)CN peptide.

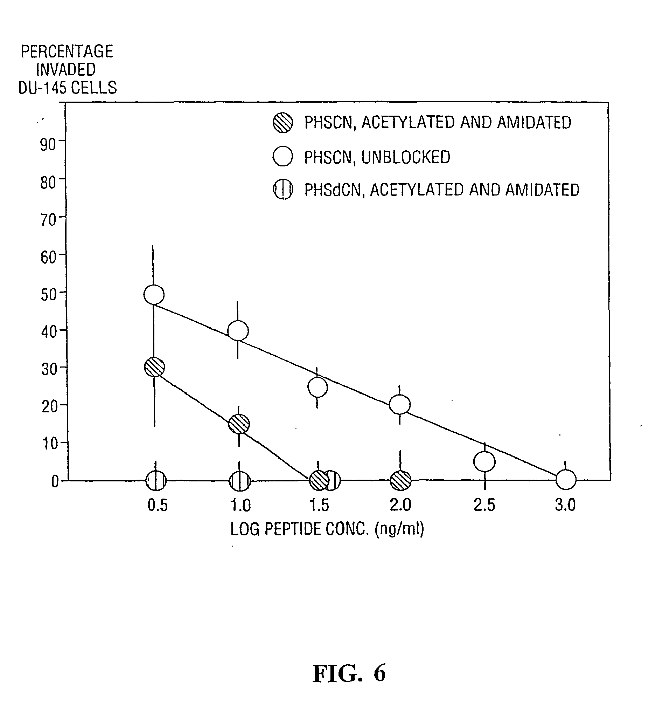

Figure 6 is a graph showing the results of inhibiting serum-induced human cancer cell invasion with varying concentrations of the PHSCN peptide, as well as PHSCN peptide that has been chemically modified with protecting groups and PHSCN peptide wherein an L-amino acid has been replaced with the D-isomer.
Figure 7 is a graph showing the results of inhibiting serum-induced human cancer cell invasion with varying concentrations of non-peptide compounds in comparison to the PHSCN peptide, as well as PHSCN peptide that has been chemically modified with protecting groups and PHSCN peptide wherein an L-amino acid has been replaced with the D-isomer.
Figure 8 is a schematic showing structural relationships between PHSCN and the non-peptide compounds of Figure 7.
Figure 9 is a graph showing the percentages of invaded neonatal fibroblasts, corresponding to various fragments of the plasma fibronectin cell binding domain, after placement on an invasion substrates. The 120 kDa and 39 kDa fragments contain the PHSRN sequence. The 11.5 kDa fragment does not. These fragments lack the α5β1 integrin binding site in the IIICS region.
Figure 10 is a graph presenting a dose response curve relating concentration of peptides containing the amino acid sequence PHSRN to fibroblast invasion into an invasion substrate.
Figure 11 is a graph presenting a dose response curve relating concentration of peptides containing the amino acid sequence PHSRN to keratinocyte invasion into an invasion substrate.
Figure 12 is a graph presenting a dose response curve relating concentration of peptides containing the amino acid sequence PHSRN to human mammary or prostate epithelial cell invasion into an invasion substrate.
Figure 13 is a graph presenting the inductive effect of peptides containing the amino acid sequence PHSRN on mouse muscle satellite cell invasion into an invasion substrate.
Figure 14 is a graph showing that increasing concentrations of the 120 kilodalton (kDa) chymotryptic fragment of fibronectin stimulate the invasion of SU-ECM invasion substrates by normal human microvascular cells.
Figure 15 is a is a graphs showing that plasma from rats growing MATLyLu tumor cells induces the invasion of normal microvascular cells into SU-ECM.
Figure 16 is an immunoblot showing the presence of fibronectin fragments of approximately 39kDa in the plasma of rats growing MATLyLu tumors.
Figure 17 presents the amino acid sequence of the invasion inducing approximately 39 kDa fragment of fibronectin described in the present application.

### DEFINITIONS

The term "inducing agent" refers to any compound or molecule which is capable of causing (directly or indirectly) the invasion of cells in a substrate. Thus, invasion inducing agents are defined functionally. This function can be readily assessed by using the invasion substrates and assays of the present invention (described below). "Inducing agents" include, but are not limited to, PHSRN-containing peptides and related peptides (see below).

The term "receptors" refers to structures expressed by cells and which recognize binding molecules (*e.g.*, ligands).

The term "antagonist" refers to molecules or compounds which inhibit the action of a "native" or "natural" compound (such as fibronectin). Antagonists may or may not be homologous to these natural compounds in respect to conformation, charge or other characteristics. Thus, antagonists may be recognized by the same or different receptors that are recognized by the natural compound. "Antagonists" include, but are not limited to, PHSCN-containing peptides and related peptides (see below).

The term "host cell" or "cell" refers to any cell which is used in any of the screening assays of the present invention. "Host cell" or "cell" also refers to any cell which either naturally expresses particular receptors of interest or is genetically altered so as to produce these normal or mutated receptors. Cells can be transfected with nucleic acid encoding a gene of interest (*i.e.* a gene encoding a particular protein, including but not limited to proteins that are therapeutic). The present invention contemplates that the peptides (and derivatives and mimetics) of the present invention are useful to facilitate and enhance the process of introducing nucleic acid into cells.

The present invention also contemplates homo-cysteine, which is identified as "hC".

The term "subject" refers to both humans and animals.

The term "fibronectin-derived peptide" means a peptide that is smaller than the intact fibronectin protein but that has sequence identical (in one embodiment having an identity of at least 80% and in a preferred embodiment having a identity of at least 90%) to a portion of the natural fibronectin sequence. For example, the peptide PHSRN has a sequence that exists in a portion of the natural fibronectin; the peptide PHSCN, while not existing as a portion of the natural sequence is, by this definition, a fibronectin-derived peptide. Typically, the peptide will be between four and one hundred amino acids (although larger fragments of fibronectin are possible, including but not limited to fragments wherein additional non-fibronectin amino acid sequences have been added to the peptide). A preferred fibronectin-derived peptide is one lacking the RGD motif of fibronectin. In yet another embodiment, said peptide lacks the motif which binds the α5β1 receptor.

The term "peptide derivative" refers to compound having an imino group (-NH-), and more particularly, a peptide bond. Peptides may be regarded as substituted amides. Like the amide group, the peptide bond shows a high degree of resonance stabilization. The C-N single bond in the peptide linkage has typically about 40 percent double-bond character and the C=O double bond about 40 percent single-bond character. Peptide derivatives include (but are not limited to) "peptide analogs" which are herein defined as compounds that can be incorporated into polypeptide chains in place of the corresponding natural amino acids by the natural enzymes (i.e. incorporated by aminoacyl-tRNA synthetases. Examples of such analogues include (but are not limited to) p-fluorophenylalanine (an analog of phenylalanine) and ethionine and norleucine (analogs of methionine).

"Protecting groups" are those groups which prevent undesirable reactions (such as proteolysis) involving unprotected functional groups. In one embodiment, the present invention contemplates that the protecting group is an acyl or an amide. In one embodiment, the acyl is acetate. In another embodiment, the protecting group is a benzyl group. In another embodiment, the protecting group is a benzoyl group. The present invention also contemplates combinations of such protecting groups.

The terms "imageing cancer" and "imageing of cancer" refer to detecting the presence of cancer by differentially labelling cancer as compared to non-cancerous surrounding tissues and more prefferntially, defining the boundries of cancer tissue in relation to non-cancerous tissue.

As used herein, the term "purified" means the separation of a compound from any naturally occuring milieu. In one example, compound is purified by the removal of one or more contaminants observed in any naturally occuring milieu.

As used herein, the terms "a subject suspected of having cancer" refers to an animal or human that presents any clinical manefestation of cancer. These maefestations include, but are not limited to progressive weight loss, localized pain, inflamation, anemias, edema, and cachexia.

As used herein, the term "fluid sample" refers to samples taken from whole blood, blood plasma, blood serum, extravascular fluid, cerebral spinal fluid, lymph, interstitial fluid, pleural fluid, prostatic fluid, sucular fluid, ventricular fluid, synovial fluid, and stool.

As used herein, the term "metastatic disease" refers to the pathological shifting of a disease or its local manifestations from one part of the body to another. As an example cancer, in some forms, presents as a metastatic disease.

As used herein, "MATLyLu cells" or "MATLyLu tumor cells" refer to an immortalized rat metastatic prostate carcinoma (*i.e*. the carcinoma originated in the prostate of a rat and metastasized to another location).

As used herein, "tumor" refers to any swelling or tumefaction produced by neoplastic growth.

As used herein, "cancer" refers to various types of malignant neoplasms, most of which invade surrounding tissues, that may metastasize to several sites and are likely to cause death of a subject unless adequately treated.

As used herein, "cancerous tissue" refers to a plurality of cells undergoing early, intermediate or advanced stages of multi-step neoplastic progression as previously described [H.C. Pitot (1978) in "Fundamentals of Oncology," marcel Dekker (Ed.), New York pp 15-28]. The features of early, intermediate and advanced stages of neoplastic progression have been described using microscopy. Cancer cells at each of the three stages of neoplastic progression generally have abnormal karyotypes, including translocations, inversion, deletions, isochromosomes, monosomies, and extra chromosomes. A cell in the early stages of malignant progression is referred to as "hyperplastic cell" and is characterized by dividing without control and/or at a greater rate than a normal cell of the same cell type in the same tissue. Proliferation may be slow or rapid but continues unabated. A cell in the intermediate stages of neoplastic progression is referred to as a "dysplastic cell." A dysplastic cell resembles an immature epithelial cell, is generally spatially disorganized within the tissue and loses its specialized structures and functions. During the intermediate stages of neoplastic progression, an increasing percentage of the tissue becomes composed of dysplastic cells. "Hyperplastic" and "dysplastic" cells are referred to as "pre-neoplastic" cells. In the advanced stages of neoplastic progression a dysplastic cell becomes a "neoplastic" cell. Neoplastic cells are typically invasive *i.e*., they either invade adjacent tissues, or are shed from the primary site and circulate through the blood and lymph to other locations in the body where they initiate secondary cancers. The terms "cancer," "neoplasia" or "neoplasm" refer to a plurality of cancer cells.

The term "epithelial cell" refers to a cuboidal-shaped, nucleated cell which is generally located on the surface of a tissue. A layer of epithelial cells generally functions to provide a protective lining and/or surface that may also be involved in transport processes. An epithelial cell is readily distinguished from a non-epithelial cell (*e.g.*, muscle cell, nerve cell, etc.) using histological methods well known in the art.

As used herein, "tissue" refers to a collection of similar cells and the intercellular substances around them.

As used herein, "suspected cancer" refers to single or multiple tissues suspected of comprising a neoplastic growth.

As used herein, "metastatic growth" refers to any tissue mass pathologically shifted, from a primary site, to a remote part of a body.

As used herein, "microvascular cells" refers to any cell associated with the venous, arterial, or lymphatic vasculature of a subject.

As used herein, "rat equivalents" refers to the body mass of any non-rodent subject divided by the average weight of a mature rat (*e.g.* approximately 200 grams).

As used herein, "pathological sample" refers to tissue suspected of exhibiting any manifestation of a disease that is harvested from a subject at autopsy or during a surgical procedure.

As used herein, the term "non-palpable" refers to the inability of a human sensor apparatus (e.g. finger, hand, arm, toe, foot, or leg) to detect a mass disposed on or under a layer of skin, a mucus membrane, layer of muscle or bone.

As used herein, the term "malignant" (as in a "malignant neoplasm") refers to a neoplasm that does invade and metastasize (in contrast to a benign neoplasm, which is incapable of metastasis).

As used herein, the term "carcinoma" refers to a malignant neoplasm that arose in epithelial tissue.

### DESCRIPTION OF THE INVENTION

The present invention provides compounds and methods useful in the diagnosis of cancer and the imaging of a cancerous tissue.

### I. Diagnosis Of Cancer

As a prelude to metastasis, it is believed that cancer cells proteolytically alter basement membranes underlying epithelia or the endothelial linings of blood and lymphatic vessels, invade through the defects created by proteolysis, and enter the circulatory or lymphatic systems to colonize distant sites. During this process, the secretion of proteolytic enzymes is coupled with increased cellular motility and altered adhesion. After their colonization of distant sites, metastasizing tumor cells proliferate to establish metastatic nodules.

### A. Inducing Agents

It is not intended that the present invention be limited by the nature of the agent that causes or induces cells to invade the fibronectin-free substrates of the present invention. Such agents can be identified functionally by simply adding them to the cell culture and measuring the extent of invasion. In one embodiment of the present invention, identification of these inducing agents in a fluid sample from a human patient forms the basis for a positive diagnosis for the presence of a tumor *in vivo.*

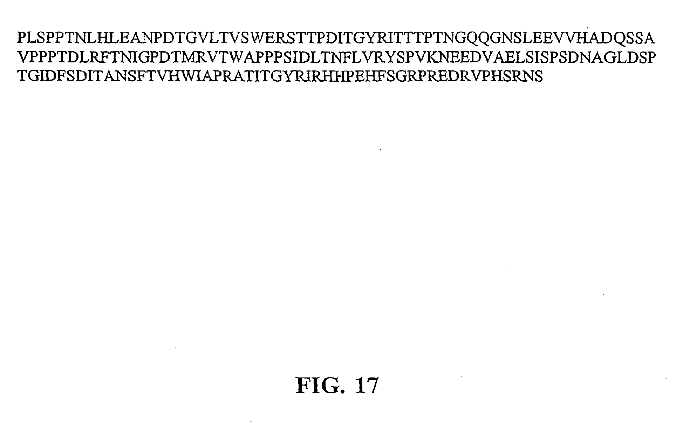

In one embodiment, the invasion inducing agent is intact fibronectin. In another embodiment, the invasion-inducing agent comprises a peptide derived from fibronectin. In a preferred embodiment, the fibronectin fragments comprise proteins, containing the PHSRN motif, with an observed molecular weight of 35kDa to 40kDa. In another preferred embodiment the fibronectin fragment comprises a peptide with an observed molecular weight of approximately 39kDa. In a more preferred embodiment the fibronectin fragment comprises the amino acid sequence set out in Figure 17.

While not limited to any mechanism, it is believed that cells exposed to invasion-inducing agents in this manner are potentially rendered capable of invading the substrate. Again, while not limited to any mechanism, it is believed that the invasion inducing agent comprising the sequence PHSRN binds to the α5β1 receptor on the cancer cell and thereby induces invasion of the substrate. In this regard, the present invention provides a method of detecting cancer: a) providing: i) cells expressing the α5β1 receptor, ii) a fibronectin-free substrate, and iii) a fluid sample collected from a patient; b) culturing (or least maintaining) said cells in serum-free culture media on said substrate in the presence of said fluid sample collected from a patient; and d) measuring the extent of cell invasion of said substrate. In one embodiment, the cells are myoblasts and neuroblasts. In a preferred embodiment the cells are normal epithelial cells or fibroblasts.

The present invention contemplates a screening assay (in the presence and absence of serum) utilizing the binding activity of fibronectin-derived peptides. In one embodiment, an inducible microvascular cell line is placed in tissue culture on a fibronectin-free substrate. The microvascular cells will be induced (under ordinary conditions) by the fibronectin-derived peptide to invade the substrate.

In one embodiment, the invasion-inducing agent comprises a peptide derived from fibronectin. In a preferred embodiment, said peptide comprises the sequence PHSRN. Of course, the peptide may be larger than five amino acids; indeed, the peptide fragment of fibronectin may contain hundreds of additional residues (*e.g.*, five hundred amino acids). One such larger peptide is set forth in U.S. Patent 5,492,890 (hereby incorporated by reference). In one embodiment, the PHSRN-containing peptide is less than one hundred amino acids in length and lacks the RGD sequence characteristic of fibronectin. A variety of PHSRN-containing peptides are contemplated, including the PHSRN peptide itself and related peptides where additional amino acids are added to the carboxyl terminus, including (but not limited to) peptides comprising the sequence: 1) PHSRN, 2) PHSRNS, 3) PHSRNSI, 4) PHSRNSIT, 5) PHSRNSITL, 6) PHSRNSITLT, 7) PHSRNSITLTN, 8) PHSRNSITLTNL, 9) PHSRNSITLTNLT, 10) PHSRNSITLTNLTP, and 11) PHSRNSITLTNLTPG. Alternatively, PHSRN-containing peptides are contemplated where amino acids are added to the amino terminus, including (but not limited to) peptides comprising the sequence: 1) PEHFSGRPREDRVPHSRN, 2) EHFSGRPREDRVPHSRN, 3) HFSGRPREDRVPHSRN, 4) FSGRPREDRVPHSRN, 5) SGRPREDRVPHSRN, 6) GRPREDRVPHSRN, 7) RPREDRVPHSRN, 8) PREDRVPHSRN, 9) REDRVPHSRN, 10) EDRVPHSRN, 11) DRVPHSRN, 12) RVPHSRN, and 13) VPHSRN. Finally, the present invention contemplates PHSRN-containing peptides where amino acids are added to both the amino and carboxyl termini, including (but not limited to) peptides comprising the sequence PEHFSGRPREDRVPHSRNSITLTNLTPG, as well as peptides comprising portions or fragments of the PHSRN-containing sequence PEHFSGRPREDRVPHSRNSITLTNLTPG.

Peptides containing variations on the PHSRN motif are contemplated. For example, the present invention also contemplates PPSRN-containing peptides for use in the above-named assays. Such peptides may vary in length in the manner described above for PHSRN-containing peptides. Alternatively, PPSRN may be used as a peptide of five amino acids.

Similarly, peptides comprising the sequence -HHSRN-, -HPSRN-, -PHTRN-,-HHTRN-, -HPTRN-, -PHSNN-, -HHSNN-, -HPSNN-, -PHTNN-, -HHTNN-, -HPTNN-,-PHSKN-, -HHSKN-, -HPSKN-, -PHTKN-, -HHTKN-, -HPTKN-, -PHSRR-, -HHSRR-,-HPSRR-, -PHTRR-, -HHTRR-, -HPTRR-, -PHSNR-, -HHSNR-, -HPSNR-, -PHTNR-,-HHTNR-, -HPTNR-, -PHSKR-, -HHSKR-, -HPSKR-, -PHTKR-, -HHTKR-, -HPTKR-,-PHSRK-, -HHSRK-, -HPSRK-, -PHTRK-, -HHTRK-, -HPTRK-, -PHSNK-, -HHSNK-,-HPSNK-, -PHTNK-, -HHTNK-, -HPTNK-, -PHSKK-, -HHSKK-, -HPSKK-, -PHTKK-,-HHTKK-, or -HPTKK- are contemplated by the present invention. Such peptides can be used as five amino acid peptides or can be part of a longer peptide (in the manner set forth above for PHSRN-containing peptides).

In another embodiment, the present invention contemplates an inducing agent comprising the amino acid sequence X₁X₂X₃X₄X₅, wherein X₁ is an amino acid selected from the group consisting of proline, glycine, valine, histidine, isoleucine, phenylalanine, tyrosine, and tryptophan, and X₂ is an amino acid selected from the group consisting of histidine, proline, tyrosine, asparagine, glutamine, arginine, lysine, glycine, phenylalanine, and tryptophan, and X₃ is an amino acid selected from the group consisting of serine, threonine, alanine, tyrosine, leucine, histidine, asparagine, glycine and glutamine, and X₄ is an amino acid selected from the group consisting of arginine, lysine, and histidine, and X₅ is an amino acid selected from the group consisting of asparagine, glutamine, serine, threonine, histidine, glycine and tyrosine.

It is also not intended that the present invention be limited by the particular cells used to evaluate induction of invasion into a given substrate. In addition, a variety of tumor cells (for both positive and negative controls) are contemplated (including but not limited to the cells set forth in Table 1 below).

While an understanding of the mechanisms involved in metastatic cancer is not necessary to the successful practice of the present invention, it is believed that tumor cell invasion of basement membranes occurs at several points in the metastatic cascade: (1) when epithelial tumor cells (such as those of breast and prostate cancers) leave the epithelium and enter the stroma, (2) when tumor cells enter the circulatory or lymphatic systems, and (3) when tumor cells leave the circulatory or lymphatic systems to invade distant sites. Thus, intervention in the *induction* of tumor cell invasiveness by using a PHSRN antagonist, such as the PHSCN peptide, to block tumor cell receptors for this sequence is contemplated as a method for selectively labeling /imaging tumor cells

One advantage of this strategy is that leukocytes are the only normal cells known to invade tissues routinely to carry out their functions, and relatively few leukocytes are invasive at a given time. Thus, relatively small doses of an anti-invasion antagonist which blocks the binding of PHSRN to its receptor are required to label / image tumor cells. Other than some immunodepression, there should be relatively few side effects associated with administration of a tumor binding protein in a method to label/image tumor cells *in vivo.* The lack of debilitating side effects expected from labeling / imaging technique means these techniques could be used before, during, and after surgery to diagnose (and subsequently confirm the removal) of a mass of tumor cells.

The IKVAV sequence of laminin, a prevalent insoluble protein of the extracellular matrix, is known to stimulate liver colonization by metastatic human colon cancer cells in athymic mice [*see* Bresalier et al., Cancer Research 55:2476 (1995)]. Since IKVAV, like PHSRN, contains a basic amino acid (K) which, by virtue of its positive charge, might also function to displace a divalent cation from its integrin receptor and stimulate invasion,the present invention contemplates applying the strategy of developing anti-invasion antagonists to the IKVAV sequence of laminin.

**TABLE 1**

| Designation And Origin Of Human Cell Lines And Strains¹ | |
|---|---|
| **Origin** | **Cell Lines or Strains** |
| Colonic carcinoma | SW1116, HCT116, SKCO-1, HT-29, KM12C, KM12SM, KM12L4, SW480 |
| Pancreatic carcinoma | BxPC-3, AsPC-1, Capan-2, MIA PaCa-2, Hs766T |
| Colon adenoma | VaCo 235 |
| Lung carcinoma | A549 |
| Prostate carcinoma | PC-3, DU-145 |
| Breast carcinoma | 009P, 013T, SUM-52 PE |
| Lymphoma | Daudi, Raji |
| Breast epithelium | 006FA |
| Diploid fibroblast | HCS (human corneal stroma), MRC-5 |

| | |
|---|---|
| The SW1116, HT-29, SW480, Raji lymphoblastoid cells, and the pancreatic lines are obtained from the American Type Culture Collection. | |

To the extent an inducing agent is detected, according to the above referenced methods, in a fluid sample from a patient; the patient is considered at risk for having a tumor *in vivo.* In this regard the present invention, in one embodiment, is contemplated to be a high throughput assay for the detection of non-palpable tumors.

### B. Assays For Detecting Tumors

In a preferred embodiment, the present invention provides compositions and methods for the detection of a non-palpable tumor mass. Such early detection assays would allow for early therapeutic interventions which would decrease the mortality and morbidity associated with the, untreated, progression of many cancers. Various assay systems (which may be applied separately or in tandem) are contemplated for use in the method of the present invention to detect tumor cells.

### 1. Fibronectin-Depleted Substrates

In one assay system, the present invention contemplates using fibronectin-depleted substrates. These are substrates that originally contain fibronectin that are treated according to the methods of the present invention (see below) to remove fibronectin. It is not intended that the present invention be limited by the nature of the original substrate; such fibronectin-containing substrates suitable for treatment and depletion include: i) complex substrates containing a variety of extracellular proteins and ii) less complex substrates containing fibronectin along with one or two other proteins (*e.g.,* collagen, laminin, etc.).

It is also not intended that the present invention be limited by the precise amount of fibronectin remaining after the substrate has been treated. In other words, while the methods of the present invention remove fibronectin, and in some embodiments, remove substantially all fibronectin, it is within the meaning of the term "fibronectin-depleted" substrate that a small amount of fibronectin remain in the substrate.

In one embodiment, the present invention contemplates using an extracellular matrix available commercially. For example, the present invention contemplates treating basement membrane matrices such as ECM GEL, a matrix from mouse sarcoma (commercially available from Sigma, St. Louis, Mo). However, it is not intended that the present invention be limited by the particular fibronectin-containing substrate. For example, other commercially available substrates are contemplated, such as the commonly used substrate Matrigel (available from Becton Dickinson Labware, Catalog #40234); Matrigel can be treated appropriately according to the methods of the present invention so as to render it "fibronectin-depleted" (see below). Untreated Matrigel (and similar substrates) have been used to demonstrate the importance of proteases and motility factors in the invasion and metastasis of many tumors. However, these invasion substrates are not available as serum-free substrates; thus, the regulation of tumor cell invasive behavior by serum components, such as plasma fibronectin, is a complicating factor with untreated Matrigel.

Consequently, the present invention contemplates a fibronectin-free substrate. In this embodiment, Matrigel is treated so that it is substantially fibronectin-free. The preparation of fibronectin-free Matrigel involves "panning" the Matrigel substrate on gelatin as well as "panning" the substrate on anti-fibronectin antibody (anti-human fibronectin IgG is available commercially, such as antibody from Promega Corporation, Madison, Wisconsin).

### 2. Naturally Occurring Fibronectin-Free Substrates

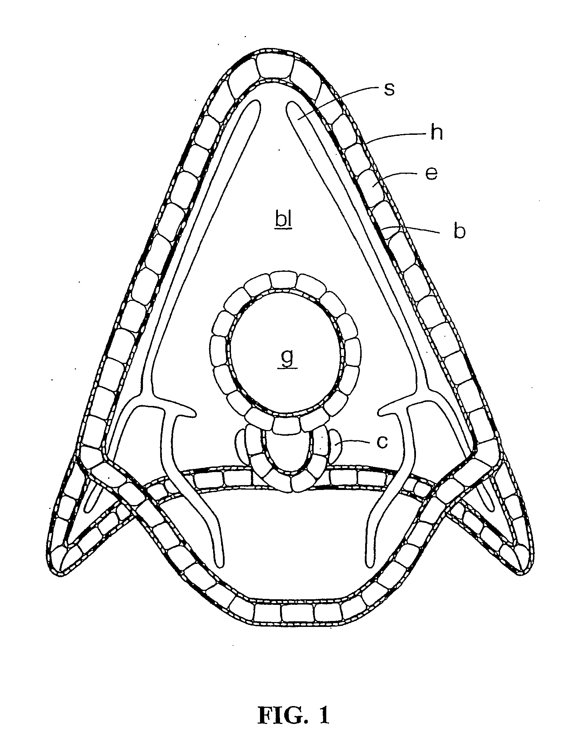

In another embodiment, the present invention contemplates substrates that are naturally free of fibronectin; such a source provides, for example, basement membranes permeable to select types of normally invasive cells, such membranes being naturally serum-free. In one embodiment, the present invention contemplates sea urchins as a source of such membranes. In this regard, the ectoderm of sea urchin embryos is one cell thick, and secretes an underlying basement membrane (see Figure 1) very similar to that of mammals. These embryos contain no circulatory or lymphatic systems; and thus, their basement membranes are serum-free. In embryos, the subectodermal basement membrane functions simultaneously as a migration substrate for several, specific mesenchymal cell types while it functions as an invasion substrate for others. Sea urchin embryo basement membranes (SU-ECM) can be prepared by mild detergent treatment as described in D. Livant *et al., Cancer Research* 55:5085 (1995).

In one example cells for the invasion assay are harvested by rinsing in Hanks' balanced salt solution, followed by brief treatment with 0.25% trypsin, 0.02% EDTA, and pelleting and resuspension in the appropriate medium with or without 5% FCS at a density of about 50,000 cells per ml. When appropriate, purified bovine plasma fibronectin (from Sigma, St. Louis, Mo), purified 120 kDa chymotryptic fragment (Gibco BRL) or PHSRN peptides (synthesized at the Biomedical Research Core Facilities of the University of Michigan) are added to the resuspended cells prior to placement of the cells on SU-ECM. In each well of a plate used for an invasion assay, SU-ECM were placed in 0.5 ml of the appropriate medium, and 0.5ml of the resuspended cells dropped on their exterior surfaces. Invasion assays were incubated 1 to 16 hours prior to assay. If some circumstances, invasion assays were fixed in phosphate-buffered saline (PBS) with 2% formaldehyde for 5 minutes at room temperature, then rinsed into PBS.

Invasion assays are coded and scored blindly by microscopic examination under phase contrast at 200- and 400-fold magnification. Each cell contacting an SU-ECM is scored for its position relative to the exterior or interior surfaces. A cell is judged to have invaded if it is located on an interior surface below the focal plane passing through the upper surface of the SU-ECM, but above the focal plane passing through its lower surface. The minimum viability of the cells in each assay is always ascertained at the time of assay by determining the fraction of spread, adherent cells on the bottom of each well scored.

An invasion frequency is defined as the fraction of cells in contact with basement membranes which are located in their interiors at the time of assay. Thus, an invasion frequency of 1 denotes invasion by 100% of the cells in contact with basement membranes. Invasion frequencies are determined multiple times for each cell type assayed. For each type of cell assayed the mean and standard deviation of the invasion frequencies were calculated.

Regardless of which of the two types of substrates are employed, the invasion substrates of the present invention are easy to prepare and give rapid, highly consistent results with a variety of cells, including but not limited to fibroblasts and keratinocytes. While not limited to any mechanism, it is believed that cells exposed to invasion-inducing agents in this manner are potentially rendered capable of invading the substrate. Again, while not limited to any mechanism, it is believed that the invasion inducing agent comprising the sequence PHSRN binds to the α5β1 receptor on the cell and thereby induces invasion of the substrate. In this regard, the present invention provides a method of treating cells comprising: a) providing i) cells expressing the α5β1 receptor, ii) a fibronectin-free substrate, and iii) one or more invasion-inducing agents; b) culturing said cells (or at least maintaining the cells briefly) in serum-free culture media on said substrate in the presence of said invasion-inducing agents; and d) measuring the extent of cell invasion of said substrate. In one embodiment, the cells are human fibroblasts.

### 3. Boyden Chamber Assays

Dermal fibroblasts obtained from punch biopsy of the skin of normal adults are grown in monolayer culture by standard techniques. Cultures are maintained in 32 oz. Brockway bottles (Brockway Glass Co., Inc. Brockway, Pa.) in a humidified atmosphere containing 5% CO₂. Eagle's minimal essential media supplemented with nonessential amino acids, ascorbic acid (50 µg/ml), NaHCO₃, and HEPES buffers (pH 7.2), penicillin (100 U/ml), streptomycin (100 µg/ml), and heat-inactivated fetal calf serum (15%) is used as maintenance media. A major portion of the cells in each bottle are harvested every 3-5 days for use in the chemotaxis assay.

Fibroblasts are dispersed routinely by pouring off the maintenance media, washing the monolayers three times with 30-40 ml of 0.015 M phosphate/0.135 M NaCl (PBS), pH 7.4, and by then adding 1.5 ml trypsin (0.25%) in PBS. During trypsinization each bottle is frequently agitated to facilitate detachment of the cells. Fibroblasts are exposed to trypsin for up to 3 min. Trypsin is inactivated by adding 10 ml maintenance media containing serum. Detached cells are collected from the Brockway bottles, centrifuged at 4°C at 300 g for 10 min, and washed two times with serum-free maintenance media. Fibroblasts are suspended at a concentration of 2.5 x 10⁵ cells/ml of serum-free media at 4°C before being used in the assay.

Blind-well-modified Boyden chemotaxis chambers (Duke University Surgical Instrument Shop, Durham, N. C.) and polycarbonate filters, 13-mm diameter, containing 8-µm pores (Wallabs Inc., San Rafeal, Calif.) are used to measure fibroblast chemotaxis. The polycarbonate filters as obtained from the manufacturer are not suitable for use in the assay because fibroblasts would adhere to and spread-out on the upper surface of the filters or migrate through the filter pores in response to a chemotactic stimulus. Fibroblasts adhere to and migrate through filters previously treated with a dilute gelatin solution so as to change the surface properties of the filters. Therefore, filters used in all experiments are treated as follows: filters are placed in wire staining, baskets (Duke University Surgical Instrument Shop), heated at 50°C in 0.5% acetic acid solution for 20 min, rinsed two times in glass-distilled water at 25°C, placed in a beaker containing gelatin in glass-distilled water (5 mg/liter) at 100°C for 1 h, dried with a hair dryer, and heated in an oven (100°C) for 1 h.

In a preferred embodiment a fluid sample (most preferably human plasma or serum) to be assayed for fibroblast chemotactic activity (CTX) is mixed with serum-free maintenance media (0.4 ml/0.35 ml). Aliquots of this mixture are placed in the lower compartment of blind-well chemotaxis chambers. Prepared polycarbonate filters (dull side up) are placed in the chambers so as to cover the filled lower compartment, and chamber caps containing the upper compartment are screwed into the chambers to effect a water tight seal around the periphery of the filter. The upper chamber compartments are then loaded with the fibroblast suspension, prepared as described above.

Loaded chambers are incubated at 37°C for 150 min in a humidified atmosphere containing 5% CO₂. After the incubation period, chambers are disassembled, filters removed, placed in staining baskets, fixed for 15 s in absolute ethanol, stained with hematoxylin, and mounted on glass cover slips. During incubation, fibroblasts responding to a chemotactic stimulus migrate from the upper filter surface through the pores and adhere to the lower filter surface. Fibroblast CTX is quantitated by counting nuclei of fibroblasts on the lower surface of the filters in 20 oil immersion fields (x 1,000). All samples are assayed in triplicate, and final activity is expressed as the mean ± SEM of the replicates.

In this model, a positive finding of Fibroblast CTX is indicates that the patient (corresponding to a given fluid sample) is likely to have a tumor.

### 4. Gold Particle Phagokinetics

Gold Particles are prepared as follows. 1.8 ml of a 14.5 mM AuCl₄H solution (J.T. Baker Chemical Co.,Phillipsburg, New Jersey) and 6 ml of a 36.5 mM Na₂CO₃ solution, both made up in quartz-distilled H₂O, are added to 11 ml quartz distilled H₂O and heated in a glass beaker over a bunsen burner. Immediately after reaching the boiling point, 1.8 ml of a 0.1% formaldehyde solution in water are quickly added. The gold particles formed immediately, producing a brownish solution which appeared clear blue in transmitted light.

22 mm X 22 mm square glass coverslips (Corning Glass Works, Science Products Division, Corning. New York, coverglass no. 1 1/2) are dipped into a 1% solution of bovine serum albumin (BSA) (Schwarz/Mann Division, Becton, Dickinson and Co., Orangeburg, New York). The BSA solution is made up in quartz-distilled water, filtered in 0.20 µm Nalgene filters (Nalgene Labware Division, Rochester, New York) and kept refrigerated. In some embodiments, however, it may be preferable to omit said BSA. The coverslip is then drained by touching a paper towel with its edge, dipped into 100% ethanol and rapidly dried in the 85°C hot air stream of a hairdryer. Then the coverslip is placed in a 3.5 cm Falcon plastic dish (Falcon Plastics, Division of BioQuest, Oxhard, California), and 2 ml of the still hot (80-90°C) gold particle suspension is layered on top.

After 45 min of incubation in the particle suspension, the now particle-coated coverslip is washed several times in culture medium (Dulbecco's modified Eagle's medium (DME) (Grand Island Biological Co., Grand Island, New York), supplemented with 10% calf serum (Microbiological Associates, Bethesda, Maryland) and placed in another 3.5 cm Falcon plastic dish containing 2 ml of DME + 10% or 20% calf serum. No special sterility precautions are required for up to 2 weeks of cell growth on particle-coated coverslips. Presumably, the hot particle suspension sterilizes the test coverslips sufficiently. For the experiments, cells are used which grow on Falcon plastic dishes for 2-3 days. 1000-2000 freshly trypsinized cells are plated in the dish, which is taken to the incubator and left there for 1 or more days. Then the coverslips are fixed for 30 min in 3.5% formaldehyde solution in phosphate-buffered saline (PBS) and mounted on microscope slides using Elvanol (DuPont Instruments, Wilmington, Delaware) as embedding medium.

The phagokinetic tracks (produced as a funtion of cell motility induced by a fluid sample from a patient) are observed in epi-darkfield illumination on a Zeiss Photomicroscope 11, using a 5x objective lens (Spindler and Hoyer, Goettingen, West Germany). The incident light emitted by a 50 watt mercury lamp (HBO 50, Osram) is passed through a green VG 9 filter and a glass prism which directed the light on the preparation, producing a large, evenly illuminated field. Pictures are taken on Kodak Plus-X 35 mm film. Scanning electron microscopy has been described elsewhere (Albrecht-Buehler and Goldman, 1976).

In this model a finding of increased phagokinetic tracks (as compared to cells not treated with fluid from a patient) indicates that the patient (corresponding to a given sample) is likely to have a tumor.

Regardless of the substrate employed, the invasion substrates of the present invention are easy to prepare and give rapid, highly consistent results with a variety of cells, including (but not limited to): a) cell lines from: i) primary and metastatic tumors, and ii) normal epithelial tissues; as well as b) cells from primary tissue samples of both tumors, their surrounding normal tissues, and neonatal melanocytes, fibroblasts, myoblasts, and keratinocytes from circumcised tissue.

In one embodiment, the present invention contemplates a method for detecting human cancer comprising: a) providing: i) a patient and ii) normal microvascular cells growing on a fibronectin-free substrate (for example, a fibronectin-depleted substrate); b) obtaining a fluid sample from said patient; c) contacting said fluid sample *ex vivo* (*i.e.,* outside the body) with said microvascular cells growing on said fibronectin-free substrate; and d) measuring the extent of microvascular cell invasion into said substrate. Preferably the microvascular cells are cultured (or at least briefly maintained) in serum-free culture media prior to testing for invasion so as to avoid introducing complicating serum factors.

### 5. Immunoblotting

In one embodiment, the present invention contemplates the resolution of proteins from a patient fluid sample by gel electrophoresis and the subsequent visualization of an approximately 39 kDa protein with a labeled antibody that binds an epitope of the PHSRN sequence. It is not intended that the present invention be limited to any specific immunoblotting protocol. However, the following is exemplary of a suitable methodology.

A precast 10% polyacrylamide (denaturing) gel is removed from the refrigerator and cooled to room temperature. Controls (+/-) and standards are prepared and adjusted to the same volume as samples. In one example, a suitable standard comprises, 5 µl Kaleidoscope Prestained standard (BioRad #1610324), 5 ng Flag-tagged protein and 40 µl 1x sample buffer.

The gel is placed into the electrophoretic apparatus. The comb is removed the inside chamber is filled with running buffer. Wells are rinsed with buffer using a pipet. The outside chamber is filled with running buffer until the buffer covers the opening on the bottom of the gel.

45 µl - 50 µl of each sample is loaded into a given well using flat loading tips. In a preferred embodiment, said sample comprises blood plasma or serum from a patient suspected of having cancer. Blank wells are filled with buffer to minimize curving of bands along the sides of the gel. The gel is run at 125 constant volts for 90 min or until blue dye front has exited gel. While gel is running, nitrocellulose, filter paper, and blotting pads are prewetted in blotting buffer.

The gel is removed from the apparatus. A gel transfer is assembled in the following orientation, starting from the bottom: (facing + electrode) top pad, filter paper, nitrocellulose, gel, filter paper, (facing - electrode) bottom pad.

The inside of the transfer chamber is filled with blotting buffer and the outside with water. The gel is transfer for 1-2 hours at 30 constant volts (Novex apparatus). After completing transfer, the apparatus is dissassebled. The transferred nitrocellulose is placed in a container with blocking solution (50 ml of 5% milk in TBS). The transferred nitrocellulose is incubated for 0.5-3.0 hours at room temperature with gentle shaking. Blocking solution is discarded and the nitrocellullose is rinsed 1x for 5 min in TBS.

The nitrocellulose is placed in a container with antibody that binds an epitope of the PHSRN sequence. In a preferred embodiment this antibody is mouse anti-human fibronectin monoclonal antibody (Chemicon International, Cat. No. MAB1926, Temecula, CA) Ml directly conjugated to HRP (Chromaprobe Inc., custom conjugated M1-HRP). The nitrocellulose is incubated (with the conjugated antibody) for 1-3 hours at room temperature, with gentle shaking. The incubation buffer comprises, 1 mM CaCl2 (20 µl 1 M CaCl2), 1% BSA (2 ml 10% BSA), 0.25 µg/ml M1-HRP (5 µl 1µg/µl M1-HRP) in 1x TBS, 0.1% Tween-20 (18 ml 1x TBS, 0.1% Tween). The blot is rinsed 1x with TBS-T Ca++ wash buffer and then washed 2x, 15 min per wash.

The nitrocellulose is removed from the wash buffer and place on saran wrap. 4 ml of solution #1 and 4 ml of solution #2 (Amersham #RPN2106) are combined. This combined mixture is immediately poured onto the surface of the nitrocellulose. The nitrocellulose is reacted with this mixture for 1 min at room temperature. The developing mixture is poured off and the nitrocellulose is enclosed in saran wrap and a fluorescent marker is placed next to the nitrocellulose. This wrapped nitrocelluse is then transferred to a film cassette. In dark room, a piece of film is placed on top of the wrapped nitrocellulose. The film is exposed for 1 min at room temperature and subsequently developed. If the signal is low, increase the exposure time is increased.

A signal (e.g. band) detected in at approximately 39kDa (vis-a-vis the molecular weight markers) indicates that the patient (corresponding to a given fluid sample) is likely to have a tumor.

### EXPERIMENTAL

The following examples serve to illustrate certain preferred embodiments and aspects of the present invention and are not to be construed as limiting the scope thereof.

In the experimental disclosure which follows, the following abbreviations apply: eq (equivalents) ; µ (micron); M (Molar); µM (micromolar); mM (millimolar); N (Normal); mol (moles); mmol (millimoles); µmol (micromoles); µmol (nanomoles); g (grams); mg (milligrams); µg (micrograms); ng (nanograms); L (liters); ml (milliliters); µl (microliters); cm (centimeters); mm (millimeters); µm (micrometers); nM (nanomolar);°C (degrees Centigrade); mAb (monoclonal antibody); MW (molecular weight); PBS (phophate buffered saline); U (units); d(days).

### EXAMPLE 1

### Production Of Fibronectin-Free Substrates

This example describes a purification approach for removal of plasma fibronectin (and/or cellular fibronectin) from a substrate (Matrigel). In this example, removal was attempted by affinity chromatography over Gelatin-Sepharose (a technique which can be used to remove plasma fibronectin from fetal calf serum).

The Gelatin-Sepharose beads were obtained from Pharmacia (Catalog# 17-0956-01). Two Kontes columns were set up with about 2 mls of Gelatin-Sepharose beads at 4 °C to prevent gelling of the Matrigel. The columns were then rinsed with about 10 column volumes of PBS to remove the preservative from the beads. The columns were drained to the top of the beads; then Matrigel was carefully added to the column. Once the Matrigel had entered the column, PBS was added to the top of the column. The Matrigel which was passed over the first column was collected and passed over the second column. The fibronectin-depleted Matrigel collected from the second column was plated on 48-well plates (150 µl/well), sterilized under a UV light for 10 minutes and incubated at 37 °C overnight. The Matrigel treated in this manner failed to form a gel at 37 °C.

### EXAMPLE 2

### Production Of Fibronectin-Free Substrates

This example describes a purification approach for removal of plasma fibronectin (and/or cellular fibronectin) from a substrate (Matrigel). In this example, removal was attempted by successive panning on gelatin. Eight wells of 24-well plate were coated with a 2% gelatin solution (the gelatin was obtained from Becton Dickinson Labware, Catalog #11868). The wells were filled with the gelatin solution which had been heated to 50 C and incubated for 3 minutes. Then the solution was removed and the wells were allowed to air dry. Following drying, the wells were thoroughly rinsed with ddH₂O followed by two rinses with PBS. The plates were again allowed to dry; thereafter they were stored at -20 °C until use. Matrigel was thawed on ice and then added to one of the wells of a gelatin-coated plate (between 800 µl and 1 ml of Matrigel was added to a well of a 24-well plate). The plate was placed in a bucket of ice in a 4°C room on an orbital shaker where the Matrigel was incubated in the well for two hours (although overnight incubation can be used). Following the incubation, the Matrigel was moved from the first well to a second well and then incubated for two hours under the same conditions. This process was repeated until the Matrigel had been incubated on all eight wells of the gelatin-coated plate.

Following the depletion of the Matrigel, it was collected in Eppendorf tubes. It was then plated on a 48-well plate (150 µl/well), sterilized under a UV light for 10 minutes and incubated at 37 °C overnight. The Matrigel formed as gel and the following day, cells were added to each well.

### EXAMPLE 3

### Production Of Fibronectin-Free Substrates

This example describes a purification approach for removal of plasma fibronectin (and/or cellular fibronectin) from a substrate (Matrigel). In this example, removal was attempted by gelatin panning followed by antibody panning.

*Anti-fibronectin antibody-coated wells:* Wells of a 24-well plate were coated with an anti-fibronectin antibody. A mouse monoclonal antibody to human fibronectin was obtained from Oncogene Science (Catalog #CP13). Each well was incubated with 1 ml of antibody at a concentration of 30 µl/ml for 2 hours at room temperature. Each well was then incubated with a solution of 3% BSA in PBS for 2 hours at room temperature. Following the two incubation periods, the wells were thoroughly washed with PBS and stored at -20 °C until use.

*Depleting Matrigel of Fibronectin:* Matrigel was panned over eight gelatin-coated wells (as described above in Example 2) to remove most of the fibronectin and its fragments. Thereafter, the Matrigel was placed in the antibody-coated wells to remove any remaining fragments of fibronectin which contain the cell-binding domain but not the gelatin-binding domain. The Matrigel was incubated in an ice bucket on an orbital shaker at 4 °C for 2 hours. Once the Matrigel was depleted, it was collected in Eppendorf tubes. The fibronectin-depleted Matrigel was plated on a 48-well plate (150 µl/well), sterilized under a UV light for 10 minutes and incubated at 37 °C overnight. The Matrigel formed a gel and the following day, cells were added to the wells.

### EXAMPLE 4

### Inducing Invasive Behavior Of Tumor Cells

In this example, the role of plasma fibronectin in inducing the invasive behaviors of metastatic breast and prostate cancer cells is demonstrated. Human breast carcinoma cell lines SUM 52 PE and SUM 44 PE were originally cultured from the pleural effusions of patients with metastatic breast cancer; and SUM 102 was cultured from a primary, microinvasive breast carcinoma (Ethier, S.P., Mahack, M.L., Gullick, W.J., Frank, T.S., and Weber, B.L. Differential isolation of normal luminal mammary epithelial cells and breast cancer cells from primary and metastatic sites using selective media. Cancer Res. 53: 627-635). The DU 145 metastatic human prostate cancer cell line was originally cultured from a brain metastasis (Stone, K.R., Mickey, D.D., Wunderli, H., Mickey, G.H., Paulsen, D.F. (1978) Isolation of a human prostate carcinoma cell line (DU 145), Int. J. Cancer 21: 274-281. These cell lines can all be maintained for at least 24 hours in serum-free conditions; thus they are ideal for use in serum-free invasion assays on SU-ECM.

Adult *Strongylocentrotus purpuratus* sea urchins were obtained from Pacific BioMarine, and their embryos were cultured to the early pluteus stage in artificial sea water at 15°C. SU-ECM were prepared from them by treatment with nonionic detergent and sterilized by dilution in the appropriate media.

Cells were harvested by rinsing in Hanks balanced salt solution, followed by brief treatment with 0.25% trypsin, 0.02% EDTA, and pelleting and resuspension in the appropriate medium with or without 5% FCS at a density of about 50,000 cells per ml. When appropriate, purified bovine plasma fibronectin (Sigma, St. Louis, Mo), purified 120 kDa chymotryptic fragment (Gibco BRL), PHSRN or PHSCN peptides (synthesized at the Biomedical Research Core Facilities of the University of Michigan), or GRGDSP or GRGESP peptides (Gibco BRL) were added to the resuspended cells prior to placement of the cells on SU-ECM. In each well of a plate used for an invasion assay, SU-ECM were placed in 0.5 ml of the appropriate medium, and 0.5ml of the resuspended cells dropped on their exterior surfaces. Invasion assays were incubated 1 to 16 hours prior to scoring. If some circumstances, invasion assays were fixed in phosphate-buffered saline (PBS) with 2% formaldehyde for 5 minutes at room temperature, then rinsed into PBS.

Invasion assays were coded and scored blindly by microscopic examination under phase contrast at 200- and 400-fold magnification. Each cell contacting an SU-ECM was scored for its position relative to the exterior or interior surfaces. A cell was judged to have invaded if it was located on an interior surface below the focal plane passing through the upper surface of the SU-ECM, but above the focal plane passing through its lower surface. The minimum viability of the cells in each assay was always ascertained at the time of assay by determining the fraction of spread, adherent cells on the bottom of each well scored.

An invasion frequency is defined as the fraction of cells in contact with basement membranes which were located in their interiors at the time of assay. Thus, an invasion frequency of 1 denotes invasion by 100% of the cells in contact with basement membranes. Invasion frequencies were determined multiple times for each cell type assayed. For each type of cell assayed the mean and standard deviation of the invasion frequencies were calculated.

The invasion-inducing sequences of plasma fibronectin were mapped to a peptide sequence 5 amino acids long, the PHSRN peptide, for both metastatic breast and prostate cancer cells. Since the PHSRN sequence is present in plasma fibronectin, a significant component of serum, eliciting the regulatory role of this sequence was only possible because of the availability of a serum-free *in vitro* invasion substrate. It should be noted that neonatal, human fibroblasts are also induced with the PHSRN peptide to invade serum-free SU-ECM. Although fibroblasts do not invade SU-ECM in the presence of serum, the 120 kDa fragment of plasma fibronectin containing the PHSRN sequence can induce fibroblast invasion equally well in the presence of serum or in its absence.

When taken together, the results of experiments showing that the PHSRN sequence of plasma fibronectin induces the invasive behaviors of both metastatic breast and prostate cancer cells, as well as that of normal fibroblasts suggest the intriguing possibility that the invasive behavior associated with tumor cell metastasis may result from defects in the regulation of the normal invasive behaviors associated with wound healing.

### EXAMPLE 5

### Improving Gelatin Depletion As Measured By Fibroblast Invasiveness

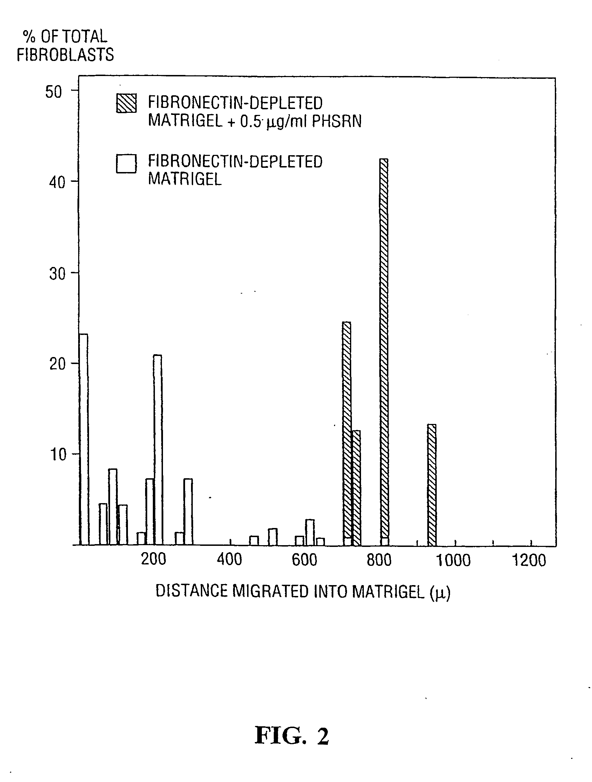

In this example, normal, neonatal fibroblasts were tested on the depleted Matrigel material prepared according to Example 3 above (*i.e.,* antibody depletion). As shown in Figure 2, panning with an antibody after gelatin depletion improved the method for removal, as measured by the reduced invasiveness of fibroblasts. On the other hand, invasiveness of the fibroblasts could be induced by the addition of the PHSRN peptide.

The success of antibody panning suggests the feasibility of removing other components by the antibody panning methods. Other serum components, such as thrombospondin, growth factors and cytokines are contemplated by the present invention for removal by the appropriate (commercially available) antibody.

### EXAMPLE 6

### Conjugation Of PHSRN-Containing Peptides

In this example, the preparation of a peptide conjugate is described. The synthetic peptide NH₂ - PHSRNC can be prepared commercially (*e.g.*, Multiple Peptide Systems, San Diego, CA). The cysteine is added to facilitate conjugation to other proteins.

In order to prepare a protein for conjugation (*e.g.*, BSA), it is dissolved in buffer (*e.g.*, 0.01 M NaPO₄, pH 7.0) to a final concentration of approximately 20 mg/ml. At the same time n-maleimidobenzoyl-N-hydroxysuccinimide ester ("MBS" available from Pierce) is dissolved in N,N-dimethyl formamide to a concentration of 5 mg/ml. The MBS solution, 0.51 ml, is added to 3.25 ml of the protein solution and incubated for 30 minutes at room temperature with stirring every 5 minutes. The MBS-activated protein is then purified by chromatography on a Bio-Gel P-10 column (Bio-Rad; 40 ml bed volume) equilibrated with 50 mM NaPO₄, pH 7.0 buffer. Peak fractions are pooled (6.0 ml).

The above-described cysteine-modified peptide (20 mg) is added to the activated protein mixture, stirred until the peptide is dissolved and incubated 3 hours at room temperature. Within 20 minutes, the reaction mixture becomes cloudy and precipitates form. After 3 hours, the reaction mixture is centrifuged at 10,000 x g for 10 min and the supernatant analyzed for protein content. The conjugate precipitate is washed three times with PBS and stored at 4°C.

From the above, it should be clear that the present invention provides a method of testing a wide variety of tumor types, and in particular identifying invasive tumors. With a means of identifying such tumors (now provided by the present invention) and distinguishing such tumors from non-invasive cancer, the physician is able to change and/or optimize therapy. Importantly, the antagonists of the present invention (and other drugs developed by use of the screening assay of the present invention) will provide treatment directed an invasive cells (and therefore associated with minimal host toxicity).

### EXAMPLE 7

### Inhibiting Invasion Of Human Breast Cancer Cells

In this example, the role of the PHSCN peptide in inhibiting the invasive behavior of metastatic breast cancer cells is demonstrated. The method of Example 4 is employed, with the addition of varying concentrations of the PHSCN peptide.

Example 4 indicates that SUM-52 cells (in medium with 5% fecal calf serum) are induced to invade the SU-ECM substrate in the presence of serum fibronectin or just the PHSRN sequence of fibronectin. Thus, the procedure in Example 4 provides a method for determining the inhibitory potential of the PHSCN peptide by comparing the number of cell invasions in the presence of the PHSCN peptide, with the number of cell invasions in the absence of the PHSCN peptide.

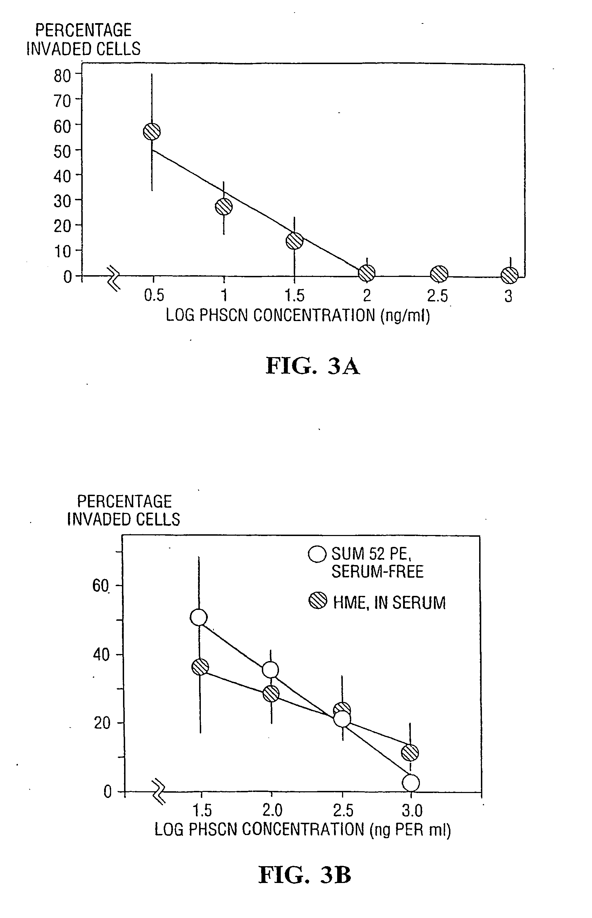

The results of adding varying concentrations of the PHSCN peptide to serum-induced metastatic SUM-52 PE breast cancer cells are presented in Figure 3A. The logs of the PHSCN peptide concentrations in ng per ml are plotted on the X axis. The percentages of invaded SUM 52 PE cells relative to the percentage invaded in the absence of the PHSCN peptide are plotted on the Y axis. Mean invasion percentages are shown with their first standard deviations. Clearly, the PHSCN peptide exhibits a substantial inhibitory affect on these cells, even at relatively low concentrations. The PHSCN peptide's inhibitory affect is further demonstrated by the fact that relatively high concentrations cause complete inhibition.

The results of adding varying concentrations of the PHSCN peptide to PHSRN-induced invasion of both metastatic SUM-52 PE breast cancer cells (in serum free media) and normal human mammary epithelial cells (in 10% FCS), are presented in Figure 3B. All invasion assays were carried out in 100 ng per ml of the PHSRN peptide to induce invasion. Again, the PHSCN peptide exhibits a substantial inhibitory affect on both cell lines at low concentrations, and almost complete inhibition at higher concentrations.

This example demonstrates the PHSCN peptide is an effective inhibitor of human breast cancer cell invasion. In this manner, the PHSCN peptide, or related sequences, are likely to provide effective therapy for human breast cancer by preventing the lethal affects of tumor cell metastasis. In addition, these data show that PHSCN selectivly binds to human breast cancer tumor cells.

### EXAMPLE 8

### Inhibiting Invasion Of Human Prostate Cancer Cells

In this example, the role of the PHSCN peptide in inhibiting the invasive behavior of metastatic prostate cancer cells is demonstrated. The method of Example 4 is employed, with the addition of varying concentrations of the PHSCN peptide.

Example 4 indicates that DU-145 cells are induced to invade the SU-ECM substrate in the presence of serum fibronectin or just the PHSRN sequence of fibronectin. Thus, the procedure in Example 4 provides a method for determining the inhibitory potential of the PHSCN peptide by comparing the number of cell invasions in the presence of the PHSCN peptide, with the number of cell invasions in the absence of the PHSCN peptide.

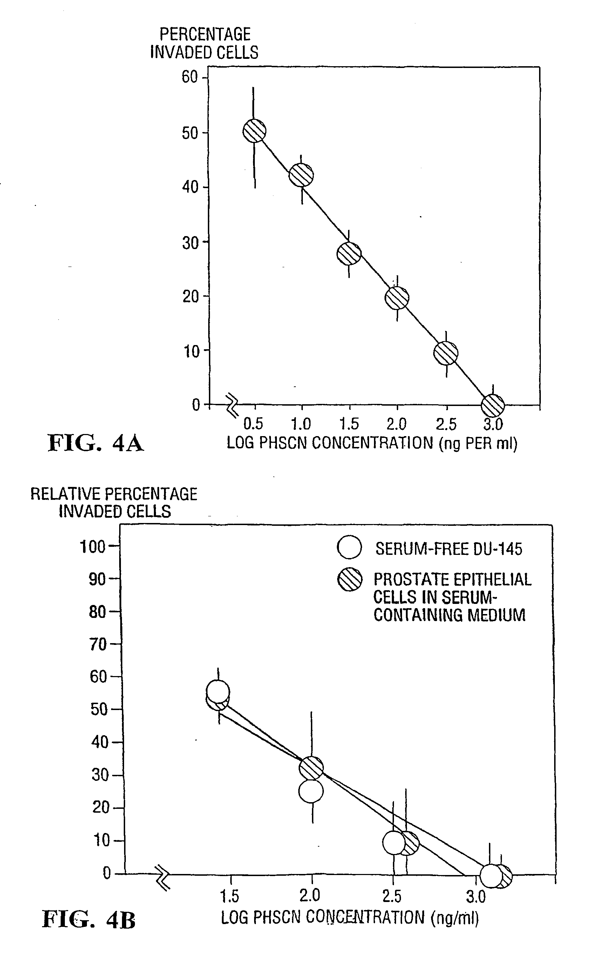

The results of adding varying concentrations of the PHSCN peptide to serum-induced invasion of metastatic DU-145 prostate cancer cells (in 10% serum) are presented in Figure 4A. The logs of the PHSCN concentrations are plotted on the X axis. The percentages of invaded DU-145 cells relative to the percentage invaded in the absence of the PHSCN peptide are plotted on the Y axis. Mean invasion percentages are shown with their first standard deviations. Clearly, the PHSCN peptide exhibits a substantial inhibitory affect on these cells, even at relatively low concentrations. The PHSCN peptide's inhibitory affect is further demonstrated by the fact that relatively high concentrations cause complete inhibition.

The results of adding varying concentrations of the PHSCN peptide to PHSRN-induced metastatic DU-145 prostate cancer cells (in serum-free medium) or to normal human prostate epithelial cells (in 10% FCS), are presented in Figure 4B. All invasion assays were carried out in 100 ng per ml of the PHSRN peptide to induce invasion. Again, the results show that the PHSCN peptide exhibits a substantial inhibitory affect on both cell lines at low concentrations, and almost complete inhibition at higher concentrations.

This example demonstrates the PHSCN peptide is an effective inhibitor of human prostate cancer cell invasion. In this manner, the PHSCN peptide may provide an effective therapy for human prostate cancer by preventing the lethal affects of tumor cell metastasis. In addition, these data show that PHSCN selectivly binds to human prostate cancer tumor cells.

### EXAMPLE 9

### Inhibiting Invasion Of Rat Prostate Cancer Cells

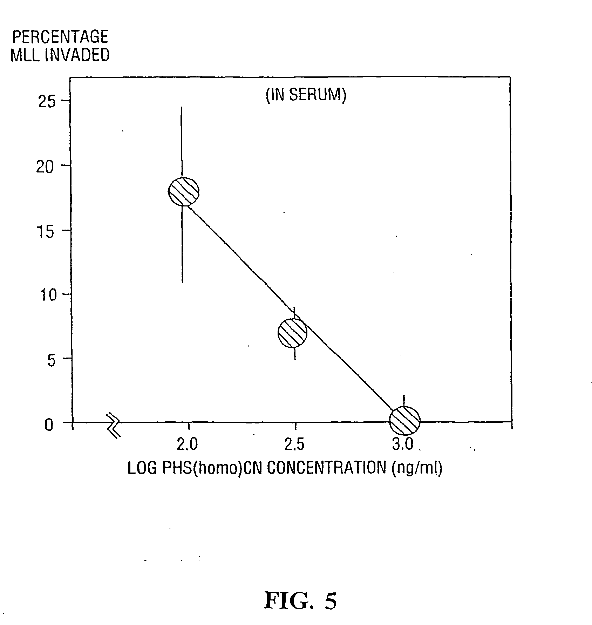

In this example, the role of a homo-cysteine containing peptide (*i.e.*, PHS(hC)N) in inhibiting the invasive behavior of rat metastatic prostate carcinoma MatLyLu (MLL) cells is demonstrated. The procedure was employed using SU-ECM substrates in 10% FCS and PHS(hC)N instead of PHSCN. The result of adding varying concentrations of the PHS(hC)N peptide to serum-induced MLL cells indicates this peptide also has an inhibitory affect on cell invasion (see Figure 5). As with the PHSCN peptide, the PHS(hC)N peptide substantially inhibits invasion at lower concentrations, and completely inhibits invasion at higher concentrations. This example demonstrates that the PHS(hC)N peptide has a similar inhibitory affect as the PHSCN peptide. In addition, these data show that PHS(hC)N selectivly binds to rat metastatic prostate carcinoma MatLyLu (MLL) cells.

### EXAMPLE 10

### Chemically Modified Peptides And Cancer Therapy

In this example, *in vitro* invasion of cancer cells into substrates is inhibited with peptides that have been chemically modified with protecting groups and peptides having protecting groups as well as the modification wherein an L-amino acid has been replaced with the D-isomer. PHSCN peptide, blocked PHSCN peptide, and blocked PHSCN peptide with a D-cysteine instead of the L-isomer, were tested (Figure 4A, 4B). The PHSCN peptide without protecting groups (*i.e.* "unblocked" PHSCN, open circles) completely inhibits the invasion of metastatic DU145 cells in medium with 10% serum at a concentration of 3 ng/ml. When PHSCN is protected from exoproteinase degradation by N-terminal acetylation and C-terminal amidation, the Ac-PHSCN-NH₂ peptide (closed circles) can completely inhibit the invasion of metastatic DU145 cells in medium with 10% serum at a concentration of approximately 1.3 ng/ml. Substitution of the D-isomer of cysteine (making Ac-PHS(dC)N-NH₂ for protection against endoproteolytic activity, striped circles) further increases the invasion-inhibitory activity of the peptide.

While not intending to limit the invention to any particular mechanism, it is believed that increases in activity may be due to proteinase resistance. Such proteases may be found in the serum or may be produced by the DU145 cells (i.e. both membrane-bound and secreted proteases). If proteinase resistance is the reason for the additional activity, this suggests that such modified peptides will have longer circulating half-lives upon administration to animals, including humans. This will permit lower dosages for therapy.

Alternatively, these changes may make the inhibitors interact with the PHSRN-binding pocket of the α5β1 receptor more effectively. That is to say, the addition of groups on the ends of the peptide may have a positioning or steric effect that is beneficial for binding.

### EXAMPLE 11

### Structure/Function Relationships And Designing Non-peptide Mimetics

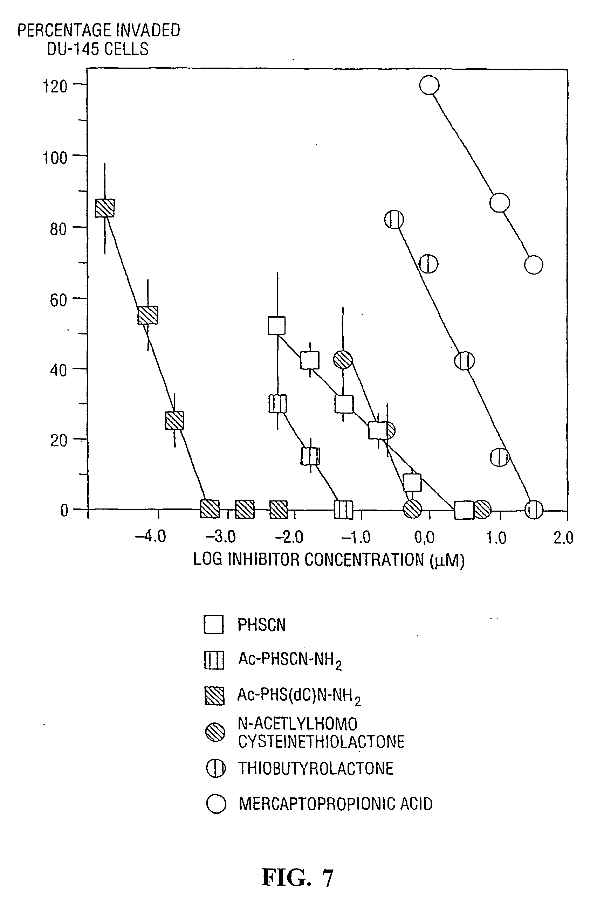

In this example, *in vitro* invasion of cancer cells into substrates is inhibited with non-peptide mimetics in comparison with chemically modified peptides (on a molar basis). The peptides of Example 10 (*i.e.* peptides chemically modified with protecting groups and peptides having protecting groups as well as the modification wherein an L-amino acid has been replaced with the D-isomer) were compared with the non-peptide mimetics DL-N-Acetylhomocysteinethiolactone, Thiobutyrolactone, and Mercaptopropionic acid, in order to investigate the structure activity relationship for invasion inhibition by interaction with the PHSRN-binding pocket of the α5β1 fibronectin receptor (Figure 7)

The PHSCN peptide without protecting groups (*i.e.* "unblocked" PHSCN, open squares) completely inhibits the invasion of metastatic DU145 cells in medium with 10% serum at a concentration of approximately 2 µM. When PHSCN is protected from exoproteinase degradation by N-terminal acetylation and C-terminal amidation, the Ac-PHSCN-NH₂ peptide (striped squares) can completely inhibit the invasion of metastatic DU145 cells in medium with 10% serum at a concentration of approximately 0.06 µM. Substitution of the D-isomer of cysteine (making Ac-PHS(dC)N-NH₂ for protection against endoproteolytic activity, closed squares) further increases very substantially the invasion-inhibitory activity of the peptide.

The non-peptide mimetic, DL-N-Acetylhomocysteinethiolactone (closed circles) fully inhibits DU145 invasion at a concentration of 0.6 µM. Thus, is about 3-fold more active than the unblocked PHSCN peptide and about 10-fold less active than blocked PHSCN. Thiobutyrolactone (striped circles) fully inhibits DU145 invasion at a concentration of 30 µM. Thus, it is 15 times less active than the unblocked PHSCN peptide and about 500-fold less active than blocked PHSCN. It is also 50-fold less active than N-acetylhomocysteinethiolactone. Mercaptopropionic acid (open circles) did not reduce DU145 invasion by more than 20% at any of the concentrations tested. Thus, it is at least 100 times less active in invasion inhibition than thiobutyrolactone, and 1500 times less active than the unblocked PHSCN peptide.

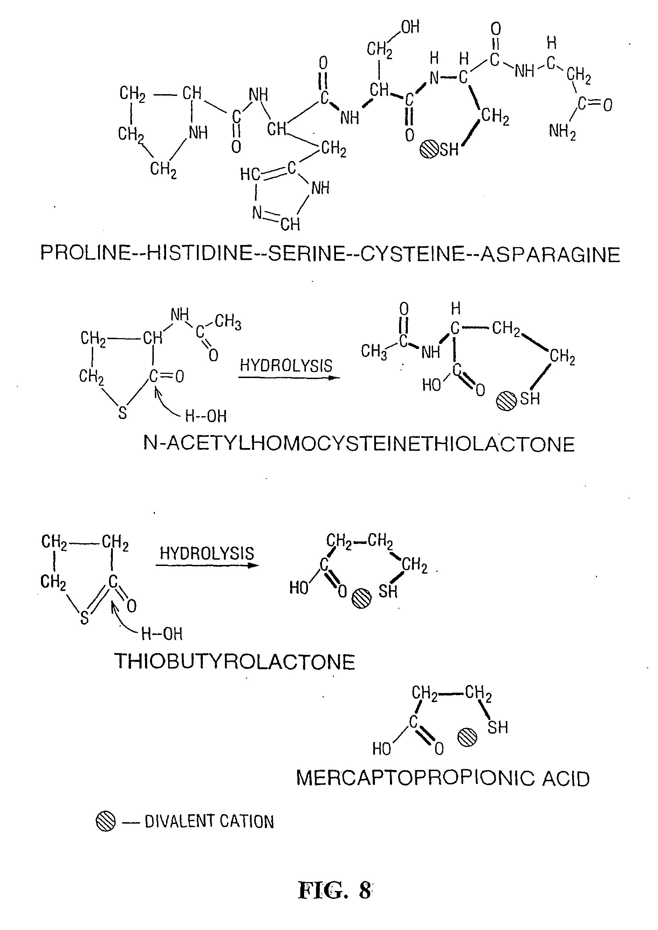

The results (Figure 7) can be examined in the context of shared structural motifs by comparing the compounds (Figure 8). The PHSCN peptide contains cysteine and the sulfhydryl group of its cysteine has the potential to form a 6-membered ring by interacting with the carbonyl oxygen of its serine residue to chelate a divalent cation known to reside in the PHSRN-binding pocket of the α5β1 integrin fibronectin receptor. The chelation of this divalent cation may prevents its displacement by the arginine of the PHSRN sequence to activate invasion. This activity may explain how PHSCN functions as a competitive inhibitor of PHSRN-induced invasion. The results with the non-peptide mimetic DL-N-Acetylhomocysteinethiolactone suggest that the amino acids surrounding the cysteine in PHSCN may contribute a 10-fold increase in its invasion-inhibiting activity, presumably by increasing its association with the PHSRN-binding pocket of the α5β1 fibronectin receptor. N-Acetylhomocysteinethiolactone has the possibility of forming a 6-membered ring, very similar to that proposed for the PHSCN invasion-inhibitory peptide, by chelating a divalent cation between its electron-rich sulfhydryl group and its carbonyl oxygen or hydroxyl group (after hydrolysis). It also has attached a nitrogen and a carbonyl group joined by a peptide linkage, which might resemble the peptide bond between the serine and histidine in the PHSCN peptide. These groups may contribute significantly to its invasion-inhibitory activity.

Thiobutyrolactone, like the homocysteinethiolactone described above, can potentially form a 6-membered ring by chelating a divalent cation between its electron-rich sulfhydryl group and its carbonyl oxygen or hydroxyl group (after hydrolysis). However, it lacks the attached peptide-linked nitrogen and carbonyl groups of the thiolactone. Thus, these attached groups may contribute a 50-fold increase in invasion inhibition.

Finally, Mercaptopropionic acid, unlike thiobutyrolactone, has the potential to form only a 5-membered ring upon interaction with the divalent cation. The poor results with this compound suggest that the presence of a 6-membered ring in the inhibitor after divalent cation chelation may increase invasion-inhibitory activity by 100-fold by stabilizing the presence of the divalent cation in the PHSRN-binding pocket. It is known that the divalent cation in this pocket is bound by 4 aspartic acids in the α5 chain of α5β1. It is interesting that the inclusion of this metal ion between the electron-rich -SH and -O or -OH groups would lead to its chelation by 6 electron-rich moities instead of 4, a much more stable configuration. The ability of the PHSCN peptide, N-Acetylhomocysteinethiolactone, and thiobutyrolactone inhibitors to form 6-membered rings as they include the divalent cation in these 6 electron-rich motifs may contribute significantly to their invasion-inhibitory activities because 6-membered organic rings are energetically favored.

The presence of an NH-COOH linked to the 6-membered ring may increase invasion-inhibitory activity by another 50-fold by mimicking the peptide linkage of the serine and histidine. This suggests that this linkage interacts with the amino acids of the PHSRN-binding pocket in a significant way. The presence of the other amino acids in the PHSCN peptide may contribute another 10-fold increase in invasion-inhibitory activity by interacting with the amino acids of the PHSRN-binding pocket of the α5β1 integrin fibronectin receptor.

### EXAMPLE 12

### Effect of Serum on PHSRN on Induction of Human Fibroblast Invasion

In this example, the invasiveness of neonatal fibroblasts into an SU-ECM invasion substrate is considered. Experiments were performed under serum-free conditions, or in medium with 10% fetal calf serum (FCS). Neither serum-free medium nor medium containing serum supported fibroblast invasion. However, consistent with the induction of metalloproteinase gene transcription by the 120 kDa fragment of plasma fibronectin (pFn) containing the cell-binding domain, the 120 kDa fragment induced fibroblast invasion in the presence or in the absence of serum.

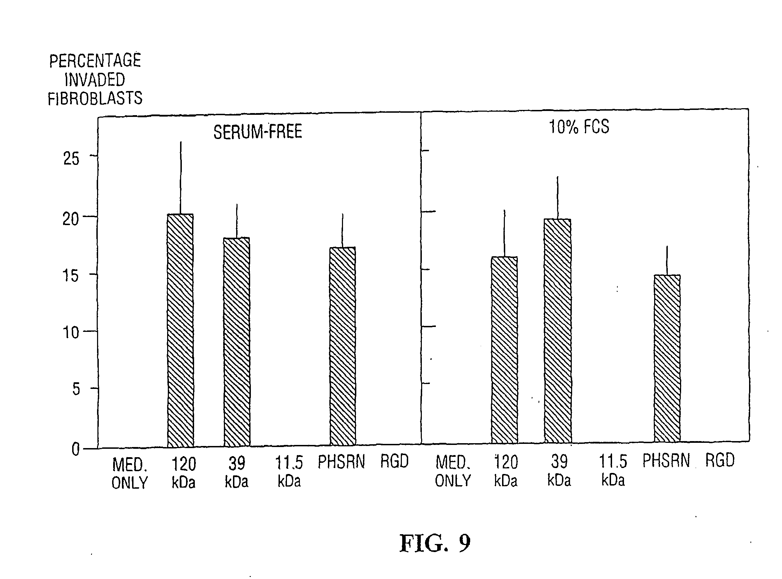

To insure the induction of invasion documented in these experiments was due to pFn sequences, and not to bound growth factors or cytokines, all of the fragments used were purified by electrophoresis on denaturing gels, followed by electroelution. Also, all fragments and sequences tested here were present in solution at a molar concentration equivalent to that of plasma fibronectin in serum. The 120 kDa cell binding domain consists of modules 2 through 11. Modules 9 and 10 are bound by the α5β1 receptor because module 9 contains the PHSRN sequence, while module 10 has the RGD sequence. Accordingly, the invasion-inducing activities of a gel-purified 39 kDa fragment containing modules 7-9 (and the PHSRN sequence) with a gel-purified 11.5 kDa fragment containing module 10 (and the RGD) sequence was considered. As can be seen in Figure 9, all of the invasion-inducing activity of the plasma fibronectin cell-binding domain appeared to map to the 39 kDa fragment bearing modules 7-9 and the PHSRN sequence. To test this observation rigorously, the PHSRN peptide, which was synthesized in a peptide synthesis CORE facility, and the GRGDS peptide, which was obtained commercially, were tested in the presence or in the absence of serum for their invasion-inducing activities. As shown in Figure 9, the PHSRN sequence contained all the invasion-stimulatory activity of the pFn cell-binding domain; and the RGD sequence had no detectable activity at the near-physiological concentrations used.

### EXAMPLE 13

Dose-response Effect Between PHSRN Concentration and Fibroblast Invasion.

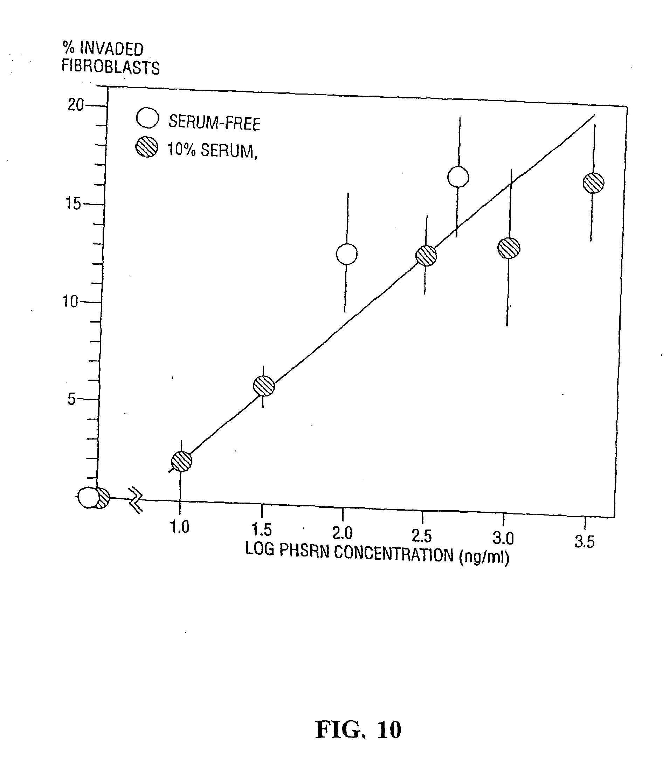

In this example, fibroblasts were induced to invade SU-ECM by concentrations of the PHSRN peptide ranging from 10 to 3000 ng per ml in the presence, or in the absence of serum. As can be seen from the dose response curves shown in Figure 10, the PHSRN peptide was able to induce fibroblast invasion in the presence of serum, which has been found to contain 40 to 80 micrograms per ml of intact plasma fibronectin, and in its absence in a similar log-linear fashion. [D.F. Mosher "Physiology of Fibronectin" Ann. Rev. Med. 35:561 (1984).]

These data suggest the metalloproteinase gene repressors produced by fibroblast α4β1 and α5β1binding of intact plasma fibronectin do not appear to bind with such high affinity that they stop PHSRN-mediated invasion induction in the presence of serum. [P. Huhtala et al. "Cooperative Signaling by α5β1 and α4β1 Integrins Regulates Metalloproteinase Gene Expression in Fibroblasts Adhering to Fibronectin" J. Cell Biol. 129: 867 (1995).] This observation is consistent with the fact that, although induced by fibronectin fragments, fibroblast invasion *in vivo* must occur in the presence of intact plasma fibronectin.

### EXAMPLE 14

### Induction of Keratinocyte Invasion by PHSRN in the Presence of Serum

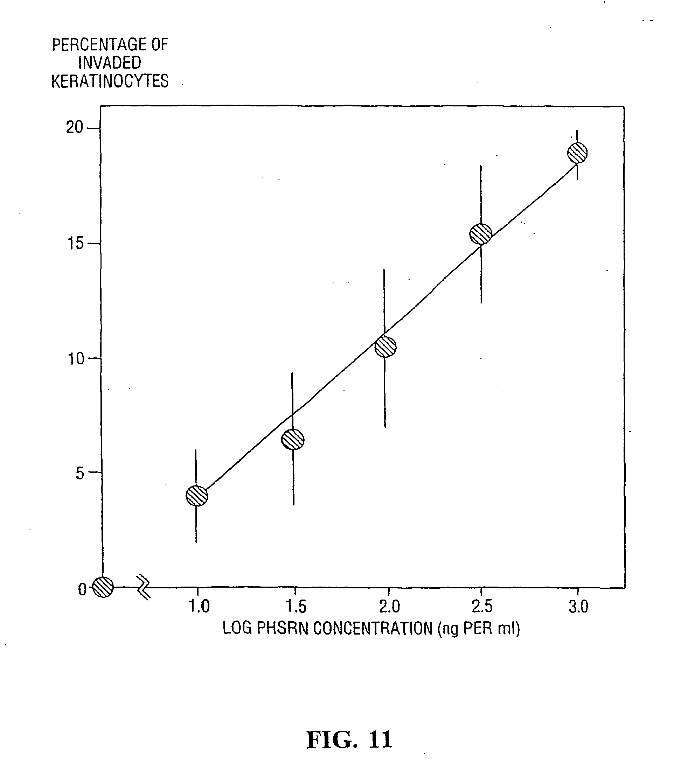

In this example the induction of normal keratinocyte invasion by PHSRN peptide, in the presence of serum, is presented. Normal neonatal keratinocytes were tested for their ability to be induced to invade SU-ECM by the PHSRN peptide. It is notable that the profile of keratinocyte invasive induction into SU-ECM by PHSRN, presented in Figure 11, is similar to the profile of invasive induction of fibroblast presented in Example 13. These data present the maximal invasion percentages for keratinocytes at a level of about 20%. Treatment of the cells (e.g. trypsin treatment) and assay conditions (e.g. time or orientation) are likely to effect this level. In any event, it is preferred that measurements are taken in the linear range.

### EXAMPLE 15

### Invasiveness of Normal Human Mammary or Prostate Epithelial Cell in Response to Induction By PHSRN in a Serum Containing Environment

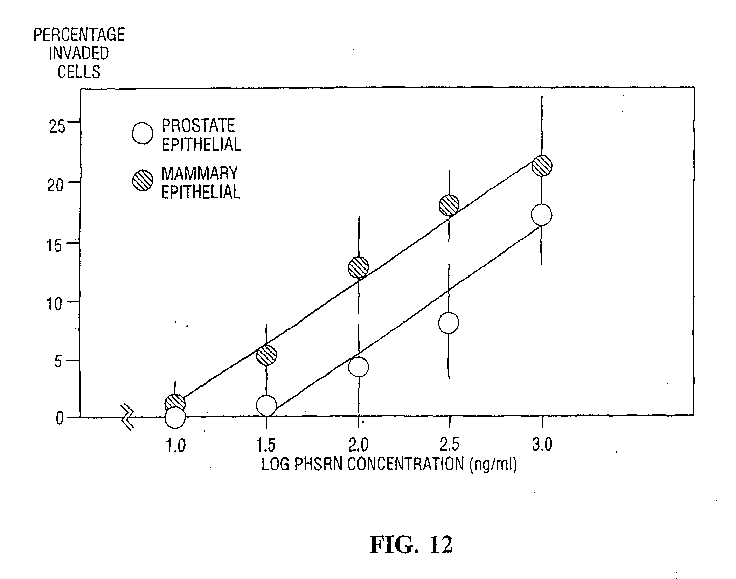

In this example, data is presented, Figure 12, demonstrating that PHSRN peptide also induces the invasive behaviors of human mammary or prostate epithelial cells. These experiments were conducted in a serum containing environment using SU-ECM as an invasion substrate. As with fibroblasts, immunostaining experiments showed that mammary and prostate epithelial cells express both the α5β1 and the α4β1fibronectin receptors (not shown); thus the ability of the α5β1 receptor to bind the PHSRN sequence on fibronectin fragments lacking the α4β1 binding site, which are generated in wounds may induce these epithelial cells to migrate into the provisional matrix or into its adjacent stroma to begin wound reepithelialization.

### EXAMPLE 16

### Invasiveness of mouse muscle satellite cells in response to induction by PHSRN in a Serum Containing Environment

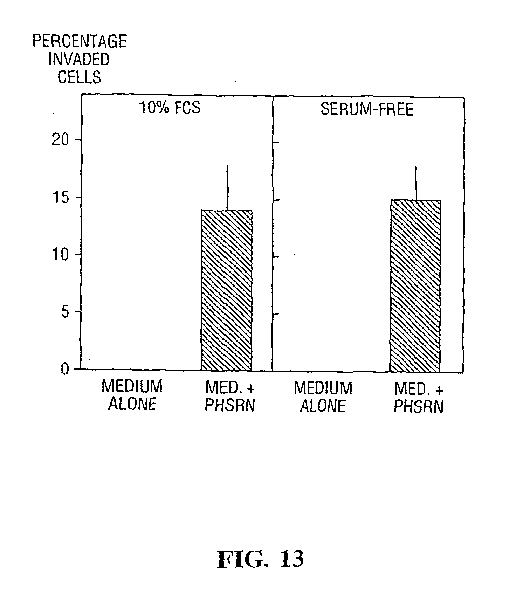

In this example, the ability of the PHSRN peptide to induce the invasive behavior of a third major tissue type, muscle cells, was considered. Mouse muscle satellite cells, which function as stem cells for muscle *in vivo,* were obtained from the laboratory of Dr. K. Kurachi (Department of Human Genetics). These cells were placed on SU-ECM invasion substrates in 1 microgram per ml of PHSRN peptide in the presence or absence of serum. As shown in Figure 13, PHSRN induced the invasion of SU-ECM by muscle satellite cells. Since muscle satellite cells are normally located inside the basement membranes surrounding the muscle fibers, in direct contact with muscle cells, and since genetically engineered muscle cells have so far failed to cross the basement membranes separating them from the muscle fibers *in vivo,* it is interesting to speculate that treatment with the PHSRN invasion-inducing peptide may induce muscle satellite cell migration into muscle *in vivo,* where these cells might resume normal function.

### EXAMPLE 17

### The cell-binding domain of plasma fibronectin stimulates SU-ECM invasion by human microvascular cells.

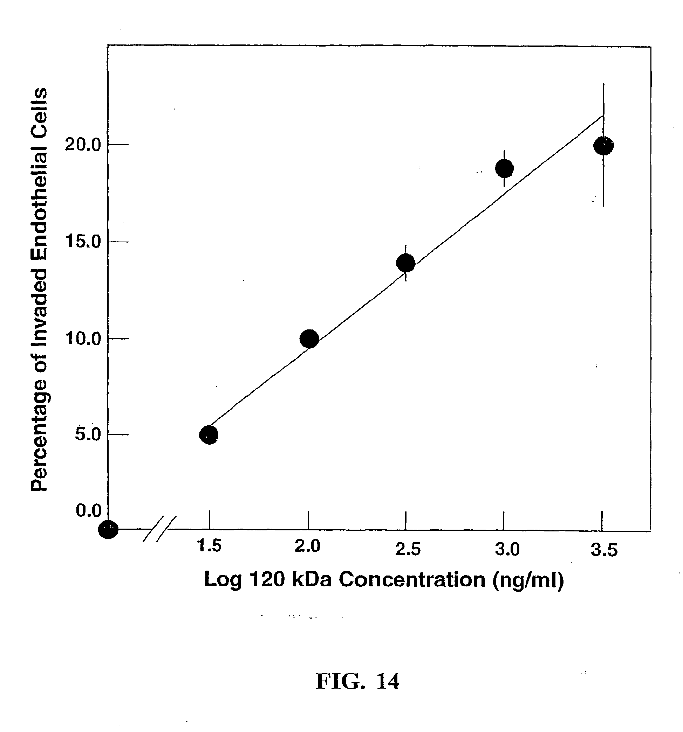

Figure 14 shows that increasing concentrations of the 120 kilodalton (kDa) chymotryptic fragment of fibronectin (available from Gibco, Inc.) stimulate the invasion of SU-ECM invasion substrates by normal human microvascular cells. The relationship between 120 kDa fragment concentration and invasion percentage appears to be approximately log-linear. SU-ECM were prepared from pluteus-stage S. purpuratus sea urchin embryos as described above. Normal microvascular cells were trypsinized from monolayer culture, pelleted, and resuspended in the appropriate concentrations of the 120 kDa fragment prior to placement on the surfaces of SU-ECM invasion substrates. All invasion assays were incubated, fixed, and scored. The log of the 120 kDa fragment concentration in nanograms per ml (ng/ml) is plotted on the X-axis. The percentage of invaded cells is plotted on the Y-axis. The percentage of invaded cells is the percentage of single cells in contact with SU-ECM, which are located on their interior rather than their exterior surfaces. The means and standard deviations of the invasion percentages from several assays are shown.

### EXAMPLE 18

### Plasma from rats with growing MATLyLu tumors stimulates microvascular cell invasion of SU-ECM, while plasma from rats without MATLyLu cells does not.

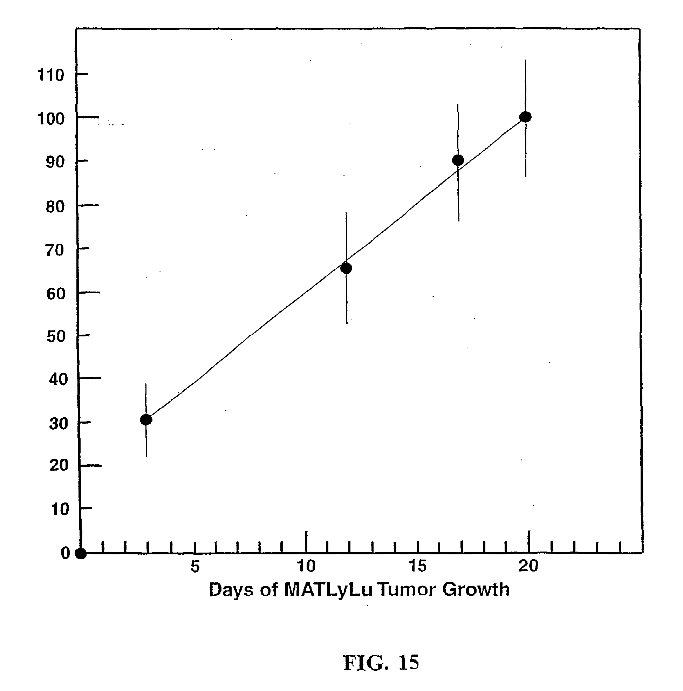

Four rats were injected 100,000 MATLyLu cells in the right flank. 0.6 ml of blood was drawn from the tail vein of each rat after 3, 12, 17, and 20 days of MATLyLu tumor growth. Tumors were palpable after 12 days of growth, but not before 11 days. Tumors were approximately 1 cm in diameter after 17 days of growth, and 2 cm after 20 days. SU-ECM *in vitro* invasion substrates were prepared from pluteus-stage *S. purpuratus* sea urchin embryos as described above. Normal microvascular cells were trypsinized from monolayer culture, pelleted, resuspended, and placed on the surfaces of SU-ECM invasion substrates in serum-free medium containing 5% plasma from rats growing MATLyLu tumors, or from rats without MATLyLu cells. All invasion assays were incubated, fixed, and scored. In Figure 15, the number of days of MATLyLu tumor growth are plotted on the X-axis. The mean percentages of invaded microvascular cells on SU-ECM invasion substrates are plotted on the Y-axis. First standard deviations are also indicated. As shown in Figure 15, medium containing 5% plasma from rats growing MATLyLu primary tumors stimulated normal microvascular cell invasion *in vitro;* whereas, medium containing 5% plasma from rats without MATLyLu cells (0 days of growth) did not.

### EXAMPLE 19

### New fragments of fibronectin are present in the plasma of rats growing MATLyLu tumors.

Plasma was obtained from 4 rats as they grew MATLyLu tumors. Plasma samples were immunoblotted with the P1H11 monoclonal anti-fibronectin antibody (Chemicon, Inc.). This antibody is known to react with the region of the pFn cell-binding domain containing the invasion -inducing PHSRN sequence.

Specifically, A 10% SDS-PAGE (29:1 acrylamide:bis acrylamide) gel was cast according to standard methods. 100 µg of rat plasma was loaded into each gel lane. The gel was run at 40 mA until the dye in the sample buffer reached the bottom of the gel. The proteing resolved on the gel were subsequently to a PVDF membrane. The membrane was blocked with 5% BSA (bovine serum albumin) in TBST buffer. This blocked membrane was subsequently probed with monoclonal anti-fibronectin antibody (1:1000 in TBST containing 5% BSA. Chemicon #MAB1926 monoclonal antibody was used which binds to 9^{th} module of Fn, where the PHSRN sequence is located). After reacting with the primary antibody, the blot was washed 2x for 5 min each with TBST. The blot was subequently reacted with a secondary antibody (1:7500 in TBST) which comprised anti-mouse IgG, conjugated to Alkaline Phosphatase (AP); Promega #S3721. The blot was then washed 3x for 5 min each with TBST, and the excess TBST blotted away. Finally, the blot was developed using 4-Cloro-3-Indolyl Phosphate/Nitro Blue Tetrazolium (Sigma, St. Louis, Mo. Catalog #B-5655)

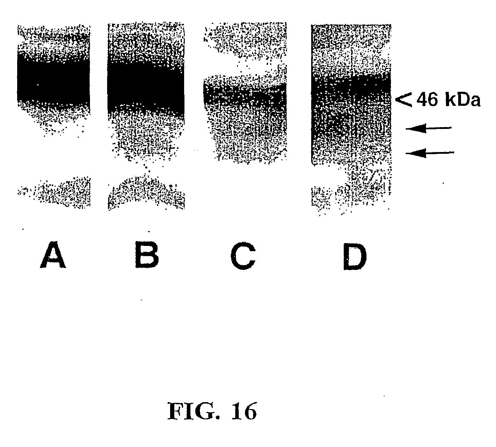

Figure 16 shows the lanes from an immunoblot containing plasma from an uninjected rat, and from a single rat at various times as it grew a MATLyLu tumor. Lane A contains the plasma from a rat that was not injected with MATLyLu cells. Lane B contains the plasma from a rat 3 days after MATLyLu cell injection. This is the stage in which invasion-inducing activity is present in the plasma, but the tumor is non-palpable. If the tumor could be detected, surgery would likely be curative at this stage. Lane C contains the plasma from a rat 12 days after MATLyLu cell injection. These fragments present an observed molecular weight of approximately 39kDa and exhibit the same epitope as the invasion-inducing 39 kDa fragment of the cell-binding domain of fibronectin. The concentration of these fibronectin fragments increased with tumor diameter and with the invasion-inducing activity of the plasma on endothelial cells *in vitro.*

This is the stage when increased invasion-inducing activity is present in the plasma, and the tumor is first palpable. Excision of a MATLyLu tumor at this stage often prevents lung metastasis. Lane D contains the plasma from a rat 20 days after MATLyLu cell injection. This is the stage when maximal invasion-inducing activity is present in the plasma, and the tumor is large (greater than 2 cm in diameter). Excision of a MATLyLu tumor at this stage can only rarely prevent lung metastasis. From the above, it should be evident that the present invention provides methods and compositions for a variety of uses. First, it should be clear that the present invention provides an cancer screening method reliable for a wide variety of tumor types, and particularly suitable for invasive tumors.

Second, compositions and methods are described for imaging tumor cells. In one embodiment this imaging comprises methods in situ visualization of a tumor mass in vivo. In another embodiment, this imaging comprises methods for the detection of tumor cells in a tissue sample in vitro.

## Claims

1. A method of detecting cancer comprising:
a) providing:
i) a fluid sample taken from a subject; and
ii) cells disposed on a fibronectin-depleted substrate, wherein said cells are disposed under conditions such that said cells do not invade said substrate;
b) contracting cells with said fluid sample; and
c) measuring the extent of invasion of said cells into said substrate, wherein the detection of invasion indicates the presence of cancer in said subject.

2. The method of claim 1, wherein said cells are microvascular cells.

3. The method of claim 2, wherein said microvascular cells are selected form the group of epithelial cells, fibroblasts, and keritinocytes.

4. The method of any one of claims 1 to 3, wherein prior to contracting said cell with said fluid said cells are cultured in serum-free media.

5. The method of any one of claims 1 to 4, wherein said fluid sample is selected from the group consisting of whole blood, plasma, and serum.

## Patentansprüche

1. Verfahren zum Detektieren von Krebs, umfassend:
a) das Bereitstellen von:
i) einer fluiden Probe entnommen von einem Subjekt; und
ii) Zellen, angeordnet auf einem Fibronektin-armen Substrat, wobei die Zellen unter Bedingungen angeordnet sind, so dass die Zellen nicht in das Substrat eindringen;
b) Inkontaktbringen von Zellen mit der fluiden Probe; und
c) Messen des Umfangs des Eindringens der Zellen in das Substrat, wobei die Detektion des Eindringens das Vorhandensein von Krebs in dem Subjekt anzeigt.

2. Verfahren nach Anspruch 1, wobei die Zellen mikrovaskuläre Zellen sind.

3. Verfahren nach Anspruch 2, wobei die mikrovaskulären Zellen ausgewählt sind aus der Gruppe von Epithelzellen, Fibroblasten und Keratinozyten.

4. Verfahren nach einem der Ansprüche 1 bis 3, wobei vor dem Inkontaktbringen der Zelle mit der fluiden Probe die Zellen in serumfreien Medien kultiviert werden.

5. Verfahren nach einem der Ansprüche 1 bis 4, wobei die fluide Probe ausgewählt ist aus der Gruppe bestehend aus Vollblut, Plasma und Serum.

## Revendications

1. Procédé de détection d'un cancer comprenant les étapes consistant à :
a) procurer :
i) un échantillon de fluide prélevé à partir d'un sujet, et
ii) des cellules disposées sur un substrat appauvri en fibronectine, lesdites cellules étant disposées dans des conditions telles que lesdites cellules n'envahissent pas ledit substrat,
b) mettre en contact les cellules avec ledit échantillon de fluide, et
c) mesurer l'étendue de l'invasion desdites cellules dans ledit substrat, la détection de l'invasion indiquant la présence d'un cancer chez ledit sujet.

2. Procédé selon la revendication 1, dans lequel lesdites cellules sont des cellules microvasculaires.

3. Procédé selon la revendication 2, dans lequel lesdites cellules microvasculaires sont sélectionnées à partir du groupe constitué de cellules épithéliales, de fibroblastes et de kératinocytes.

4. Procédé selon l'une quelconque des revendications 1 à 3, dans lequel, avant de mettre en contact lesdites cellules avec ledit échantillon fluide, lesdites cellules sont mises en culture dans un milieu exempt de sérum.

5. Procédé selon l'une quelconque des revendications 1 à 4, dans lequel ledit échantillon de fluide est sélectionné à partir du groupe constitué de sang entier, de plasma et de sérum.


### SEQUENCE LISTING

<110> Livant, Donna
<120> Compositions and Methods for the Use of Fibronectin Fragments in the Diagnosis of Cancer
<130> UM-04989
<140> 09/765,496
   <141> 2001-01-18
<160> 105
<170> Patent In version 3.1
<210> 1
   <211> 5
   <212> PRT
   <213> Artificial Sequence
<220>
   <223> Synthetic
<400> 1
   Pro His Ser Arg Asn
   1               5
<210> 2
   <211> 5
   <212> PRT
   <213> Artificial Sequence
<220>
   <223> Synthetic
<400> 2
   Cys His Ser Arg Asn
   1 5
<210> 3
   <211> 5
   <212> PRT
   <213> Artificial Sequence
<220>
   <223> Synthetic
<400> 3
   Pro Cys Ser Arg Asn
   1 5
<210> 4
   <211> 5
   <212> PRT
   <213> Artificial Sequence
<220>
   <223> Synthetic
<400> 4
   Pro His Cys Arg Asn
   1 5
<210> 5
   <211> 5
   <212> PRT
   <213> Artificial Sequence
<220>
   <223> Synthetic
<400> 5
   Pro His Ser Arg Cys
   1 5
<210> 6
   <211> 5
   <212> PRT
   <213> Artificial Sequence
<220>
   <223> Synthetic
<220>
   <221> MISC_FEATURE
   <222> (4)..(4)
   <223> The X at this position is an amino acid selected from the group c onsisting of homo-cysteine, the D-isomer of cysteine, histidine, and penicillamine.
<400> 6
   Pro His Ser Xaa Asn
   1 5
<210> 7
   <211> 5
   <212> PRT
   <213> Artificial Sequence
<220>
   <223> Synthetic
<220>
   <221> MISC_FEATURE
   <222> (1) .. (1)
<223> The X at this position is either proline, histidine, an amino aci d analogue or not an amino acid.
<220>
   <221> MISC_FEATURE
   <222> (4) .. (4)
   <223> The X at this position is an amino acid selected from the group c onsisting of the L-isomer of cysteine, the D-isomer of cysteine, homo-cysteine, histidine, and penicillarmine.
<400> 7
   Xaa His Ser Xaa Asn
   1 5
<210> 8
   <211> 5
   <212> PRT
   <213> Artificial Sequence
<220>
   <223> Synthetic
<220>
   <221> MISC_FEATURE
   <222> (1)..(1)
   <223> The X at this position is an amino acid selected from the group c onsisting of proline, glycine, valine, histidine, isoleucine, phe nylalanine, tyrosine, and tryptophan.
<220>
   <221> MISC_FEATURE
   <222> (2)..(2)
   <223> The X at this position is an amino acid selected from the group c onsisting of histidine, proline, tyrosine, glycine, asparagine, g lutamine, arginine, lysine, phenylalanine, and tryptophan.
<220>
   <221> MISC_FEATURE
   <222> (3)..(3)
   <223> The X at this position is an amino acid selected from the group c onsisting of serine, threonine, alanine, tyrosine, leucine, histi dine, asparagine, glycine, and glutamine.
<220>
   <221> MISC_FEATURE
   <222> (4) .. (4)
   <223> The X at this position is an amino acid selected from the group c onsisting of cysteine, homo-cysteine, penicillamine, histidine, t yrosine, asparagine, glutamine, and methionine.
<220>
   <221> MISC_FEATURE
   <222> (5) .. (5)
   <223> The X at this position is an amino acid selected from the group c onsisting of asparagine, glutamine, serine, threonine, histidine, glycine, and tyrosine.
<400> 8
   Xaa Xaa Xaa Xaa Xaa
   1 5
<210> 9
   <211> 5
   <212> PRT
   <213> Artificial Sequence
<220>
   <223> Synthetic
<400> 9
   Pro His Ser Cys Asn
   1 5
<210> 10
   <211> 5
   <212> PRT
   <213> Artificial Sequence
<220>
   <223> Synthetic
<400> 10
   Ile Lys Val Ala Val
   1 5
<210> 11
   <211> 5
   <212> PRT
   <213> Artificial Sequence
<220>
   <223> Synthetic
<400> 11
   Ile Cys Val Ala Val
   1 5
<210> 12
   <211> 6
   <212> PRT
   <213> Artificial Sequence
<220>
   <223> Synthetic
<400> 12
   Pro His Ser Arg Asn Ser
   1 5
<210> 13
   <211> 7
   <212> PRT
   <213> Artificial Sequence
<220>
   <223> Synthetic
<400> 13
   Pro His Ser Arg Asn Ser Ile
   1               5
<210> 14
   <211> 8
   <212> PRT
   <213> Artificial Sequence
<220>
   <223> Synthetic
<400> 14
   Pro His Ser Arg Asn Ser Ile Thr
   1               5
<210> 15
   <211> 9
   <212> PRT
   <213> Artificial Sequence
<220>
   <223> Synthetic
<400> 15
   Pro His Ser Arg Asn Ser Ile Thr Leu
   1               5
<210> 16
   <211> 10
   <212> PRT
   <213> Artificial Sequence
<220>
   <223> Synthetic
<400> 16
   Pro His Ser Arg Asn Ser Ile Thr Leu Thr
   1               5                   10
<210> 17
   <211> 11
   <212> PRT
   <213> Artificial Sequence
<220>
   <223> Synthetic
<400> 17
   Pro His Ser Arg Asn Ser Ile Thr Leu Thr Asn
   1 5 10
<210> 18
   <211> 12
   <212> PRT
   <213> Artificial Sequence
<220>
   <223> Synthetic
<400> 18
   Pro His Ser Arg Asn Ser Ile Thr Leu Thr Asn Leu
   1 5 10
<210> 19
   <211> 13
   <212> PRT
   <213> Artificial Sequence
<220>
   <223> Synthetic
<400> 19
   Pro His Ser Arg Asn Ser Ile Thr Leu Thr Asn Leu Thr
   1 5 10
<210> 20
   <211> 14
   <212> PRT
   <213> Artificial Sequence
<220>
   <223> Synthetic
<400> 20
   Pro His Ser Arg Asn Ser Ile Thr Leu Thr Asn Leu Thr Pro
   1 5 10
<210> 21
   <211> 15
   <212> PRT
   <213> Artificial Sequence
<220>
   <223> Synthetic
<400> 21
   Pro His Ser Arg Asn Ser Ile Thr Leu Thr Asn Leu Thr Pro Gly
   1 5 10 15
<210> 22
   <211> 18
   <212> PRT
   <213> Artificial Sequence
<220>
   <223> Synthetic
<400> 22
   Pro Glu His Phe Ser Gly Arg Pro Arg Glu Asp Arg Val Pro His Ser
   1               5                   10                  15
   Arg Asn
<210> 23
   <211> 17
   <212> PRT
   <213> Artificial Sequence
<220>
   <223> Synthetic
<400> 23
   Glu His Phe Ser Gly Arg Pro Arg Glu Asp Arg Val Pro His Ser Arg
   1               5                   10                  15
   Asn
<210> 24
   <211> 16
   <212> PRT
   <213> Artificial Sequence
<220>
   <223> Synthetic
<400> 24
   His Phe Ser Gly Arg Pro Arg Glu Asp Arg Val Pro His Ser Arg Asn
   1               5                   10                  15
<210> 25
   <211> 15
   <212> PRT
   <213> Artificial Sequence
<220>
   <223> Synthetic
<400> 25
   Phe Ser Gly Arg Pro Arg Glu Asp Arg Val Pro His Ser Arg Asn
   1               5                   10                  15
<210> 26
   <211> 14
   <212> PRT
   <213> Artificial Sequence
<220>
   <223> Synthetic
<400> 26
   Ser Gly Arg Pro Arg Glu Asp Arg Val Pro His Ser Arg Asn
   1 5 10
<210> 27
   <211> 13
   <212> PRT
   <213> Artificial Sequence
<220>
   <223> Synthetic
<400> 27
   Gly Arg Pro Arg Glu Asp Arg Val Pro His Ser Arg Asn
   1 5 10
<210> 28
   <211> 12
   <212> PRT
   <213> Artificial Sequence
<220>
   <223> Synthetic
<400> 28
   Arg Pro Arg Glu Asp Arg Val Pro His Ser Arg Asn
   1 5 10
<210> 29
   <211> 11
   <212> PRT
   <213> Artificial Sequence
<220>
   <223> Synthetic
<400> 29
   Pro Arg Glu Asp Arg Val Pro His Ser Arg Asn
   1 5 10
<210> 30
   <211> 10
   <212> PRT
   <213> Artificial Sequence
<220>
   <223> Synthetic
<400> 30
   Arg Glu Asp Arg Val Pro His Ser Arg Asn
   1 5 10
<210> 31
   <211> 9
   <212> PRT
   <213> Artificial Sequence
<220>
   <223> Synthetic
<400> 31
   Glu Asp Arg Val Pro His Ser Arg Asn
   1 5
<210> 32
   <211> 8
   <212> PRT
   <213> Artificial Sequence
<220>
   <223> Synthetic
<400> 32
   Asp Arg Val Pro His Ser Arg Asn
   1 5
<210> 33
   <211> 7
   <212> PRT
   <213> Artificial Sequence
<220>
   <223> Synthetic
<400> 33
   Arg Val Pro His Ser Arg Asn
   1 5
<210> 34
   <211> 6
   <212> PRT
   <213> Artificial Sequence
<220>
   <223> Synthetic
<400> 34
   Val Pro His Ser Arg Asn
   1 5
<210> 35
   <211> 28
   <212> PRT
   <213> Artificial Sequence
<220>
   <223> Synthetic
<400> 35
   Pro Glu His Phe Ser Gly Arg Pro Arg Glu Asp Arg Val Pro His Ser
   1 5 10 15
   Arg Asn Ser Ile Thr Leu Thr Asn Leu Thr Pro Gly
   20 25
<210> 36
   <211> 5
   <212> PRT
   <213> Artificial Sequence
<220>
   <223> Synthetic
   <400> 36
   Pro Pro Ser Arg Asn
   1 5
<210> 37
   <211> 5
   <212> PRT
   <213> Artificial Sequence
<220>
   <223> Synthetic
   <400> 37
   His His Ser Arg Asn
   1 5
<210> 38
   <211> 5
   <212> PRT
   <213> Artificial Sequence
<220>
   <223> Synthetic
<400> 38
   His Pro Ser Arg Asn
   1 5
<210> 39
   <211> 5
   <212> PRT
   <213> Artificial Sequence
<220>
   <223> Synthetic
<400> 39
   Pro His Thr Arg Asn
   1 5
<210> 40
   <211> 5
   <212> PRT
   <213> Artificial Sequence
<220>
   <223> Synthetic
<400> 40
   His His Thr Arg Asn
   1 5
<210> 41
   <211> 5
   <212> PRT
   <213> Artificial Sequence
<220>
   <223> Synthetic
<400> 41
   His Pro Thr Arg Asn
   1 5
<210> 42
   <211> 5
   <212> PRT
   <213> Artificial Sequence
<220>
   <223> Synthetic
<400> 42
   Pro His Ser Asn Asn
   1 5
<210> 43
   <211> 5
   <212> PRT
   <213> Artificial Sequence
<220>
   <223> Synthetic
<400> 43
   His His Ser Asn Asn
   1 5
<210> 44
   <211> 5
   <212> PRT
   <213> Artificial Sequence
<220>
   <223> Synthetic
<400> 44
   His Pro Ser Asn Asn
   1 5
<210> 45
   <211> 5
   <212> PRT
   <213> Artificial Sequence
<220>
   <223> Synthetic
<400> 45
   Pro His Thr Asn Asn
   1 5
<210> 46
   <211> 5
   <212> PRT
   <213> Artificial Sequence
<220>
   <223> Synthetic
<400> 46
   His His Thr Asn Asn
   1 5
<210> 47
   <211> 5
   <212> PRT
   <213> Artificial Sequence
<220>
   <223> Synthetic
<400> 47
   His Pro Thr Asn Asn
   1 5
<210> 48
   <211> 5
   <212> PRT
   <213> Artificial Sequence
<220>
   <223> Synthetic
<400> 48
   Pro His Ser Lys Asn
   1               5
<210> 49
   <211> 5
   <212> PRT
   <213> Artificial Sequence
<220>
   <223> Synthetic
<400> 49
   His His Ser Lys Asn
   1               5
<210> 50
   <211> 5
   <212> PRT
   <213> Artificial Sequence
<220>
   <223> Synthetic
<400> 50
   His Pro Ser Lys Asn
   1               5
<210> 51
   <211> 5
   <212> PRT
   <213> Artificial Sequence
<220>
   <223> Synthetic
<400> 51
   Pro His Thr Lys Asn
   1               5
<210> 52
   <211> 5
   <212> PRT
   <213> Artificial Sequence
<220>
   <223> Synthetic
<400> 52
   His His Thr Lys Asn
   1               5
<210> 53
   <211> 5
   <212> PRT
   <213> Artificial Sequence
<220>
   <223> Synthetic
<400> 53
   His Pro Thr Lys Asn
   1 5
<210> 54
   <211> 5
   <212> PRT
   <213> Artificial Sequence
<220>
   <223> Synthetic
<400> 54
   Pro His Ser Arg Arg
   1 5
<210> 55
   <211> 5
   <212> PRT
   <213> Artificial Sequence
<220>
   <223> Synthetic
<400> 55
   His His Ser Arg Arg
   1 5
<210> 56
   <211> 5
   <212> PRT
   <213> Artificial Sequence
<220>
   <223> Synthetic
<400> 56
   His Pro Ser Arg Arg
   1 5
<210> 57
   <211> 5
   <212> PRT
   <213> Artificial Sequence
<220>
   <223> Synthetic
<400> 57
   Pro His Thr Arg Arg
   1 5
<210> 58
   <211> 5
   <212> PRT
   <213> Artificial Sequence
<220>
   <223> Synthetic
<400> 58
   His His Thr Arg Arg
   1 5
<210> 59
   <211> 5
   <212> PRT
   <213> Artificial Sequence
<220>
   <223> Synthetic
<400> 59
   His Pro Thr Arg Arg
   1 5
<210> 60
   <211> 5
   <212> PRT
   <213> Artificial Sequence
<220>
   <223> Synthetic
<400> 60
   Pro His Ser Asn Arg
   1 5
<210> 61
   <211> 5
   <212> PRT
   <213> Artificial Sequence
<220>
   <223> Synthetic
<400> 61
   His His Ser Asn Arg
   1 5
<210> 62
   <211> 5
   <212> PRT
   <213> Artificial Sequence
<220>
   <223> Synthetic
<400> 62
   His Pro Ser Asn Arg
   1               5
<210> 63
   <211> 5
   <212> PRT
   <213> Artificial Sequence
<220>
   <223> Synthetic
<400> 63
   Pro His Thr Asn Arg
   1               5
<210> 64
   <211> 5
   <212> PRT
   <213> Artificial Sequence
<220>
   <223> Synthetic
<400> 64
   His His Thr Asn Arg
   1               5
<210> 65
   <211> 5
   <212> PRT
   <213> Artificial Sequence
<220>
   <223> Synthetic
<400> 65
   His Pro Thr Asn Arg
   1               5
<210> 66
   <211> 5
   <212> PRT
   <213> Artificial Sequence
<220>
   <223> Synthetic
<400> 66
   Pro His Ser Lys Arg
   1 5
<210> 67
   <211> 5
   <212> PRT
   <213> Artificial Sequence
<220>
   <223> Synthetic
<400> 67
   His His Ser Lys Arg
   1 5
<210> 68
   <211> 5
   <212> PRT
   <213> Artificial Sequence
<220>
   <223> Synthetic
<400> 68
   His Pro Ser Lys Arg
   1 5
<210> 69
   <211> 5
   <212> PRT
   <213> Artificial Sequence
<220>
   <223> Synthetic
<400> 69
   Pro His Thr Lys Arg
   1 5
<210> 70
   <211> 5
   <212> PRT
   <213> Artificial Sequence
<220>
   <223> Synthetic
<400> 70
   His His Thr Lys Arg
   1 5
<210> 71
   <211> 5
   <212> PRT
   <213> Artificial Sequence
<220>
   <223> Synthetic
<400> 71
   His Pro Thr Lys Arg
   1               5
<210> 72
   <211> 5
   <212> PRT
   <213> Artificial Sequence
<220>
   <223> Synthetic
<400> 72
   Pro His Ser Arg Lys
   1               5
<210> 73
   <211> 5
   <212> PRT
   <213> Artificial Sequence
<220>
   <223> Synthetic
<400> 73
   His His Ser Arg Lys
   1               5
<210> 74
   <211> 5
   <212> PRT
   <213> Artificial Sequence
<220>
   <223> Synthetic
<400> 74
   His Pro Ser Arg Lys
   1               5
<210> 75
   <211> 5
   <212> PRT
   <213> Artificial Sequence
<220>
   <223> Synthetic
<400> 75
   Pro His Thr Arg Lys
   1 5
<210> 76
   <211> 5
   <212> PRT
   <213> Artificial Sequence
<220>
   <223> Synthetic
<400> 76
   His His Thr Arg Lys
   1 5
<210> 77
   <211> 5
   <212> PRT
   <213> Artificial Sequence
<220>
   <223> Synthetic
<400> 77
   His Pro Thr Arg Lys
   1 5
<210> 78
   <211> 5
   <212> PRT
   <213> Artificial Sequence
<220>
   <223> Synthetic
<400> 78
   Pro His Ser Asn Lys
   1 5
<210> 79
   <211> 5
   <212> PRT
   <213> Artificial Sequence
<220>
   <223> Synthetic
<400> 79
   His His Ser Asn Lys
   1 5
<210> 80
   <211> 5
   <212> PRT
   <213> Artificial Sequence
<220>
   <223> Synthetic
<400> 80
   His Pro Ser Asn Lys
   1 5
<210> 81
   <211> 5
   <212> PRT
   <213> Artificial Sequence
<220>
   <223> Synthetic
<400> 81
   Pro His Thr Asn Lys
   1 5
<210> 82
   <211> 5
   <212> PRT
   <213> Artificial Sequence
<220>
   <223> Synthetic
<400> 82
   His His Thr Asn Lys
   1 5
<210> 83
   <211> 5
   <212> PRT
   <213> Artificial Sequence
<220>
   <223> Synthetic
<400> 83
   His Pro Thr Asn Lys
   1 5
<210> 84
   <211> 5
   <212> PRT
   <213> Artificial Sequence
<220>
   <223> Synthetic
<400> 84
   Pro His Ser Lys Lys
   1 5
<210> 85
   <211> 5
   <212> PRT
   <213> Artificial Sequence
<220>
   <223> Synthetic
<400> 85
   His His Ser Lys Lys
   1 5
<210> 86
   <211> 5
   <212> PRT
   <213> Artificial Sequence
<220>
   <223> Synthetic
<400> 86
   His Pro Ser Lys Lys
   1 5
<210> 87
   <211> 5
   <212> PRT
   <213> Artificial Sequence
<220>
   <223> Synthetic
<400> 87
   Pro His Thr Lys Lys
   1 5
<210> 88
   <211> 5
   <212> PRT
   <213> Artificial Sequence
<220>
   <223> Synthetic
<400> 88
   His His Thr Lys Lys
   1 5
<210> 89
   <211> 5
   <212> PRT
   <213> Artificial Sequence
<220>
   <223> Synthetic
<400> 89
   His Pro Thr Lys Lys
   1               5
<210> 90
   <211> 4
   <212> PRT
   <213> Artificial Sequence
<220>
   <223> Synthetic
<400> 90
   Pro Ser Cys Asn
   1
<210> 91
   <211> 4
   <212> PRT
   <213> Artificial Sequence
<220>
   <223> Synthetic
<400> 91
   His Ser Cys Asn
   1
<210> 92
   <211> 4
   <212> PRT
   <223> Artificial Sequence
<220>
   <223> Synthetic
<400> 92
   His Thr Cys Asn
   1
<210> 93
   <211> 4
   <212> PRT
   <213> Artificial Sequence
<220>
   <223> Synthetic
<400> 93
   Pro Thr Cys Asn
   1
<210> 94
   <211> 4
   <212> PRT
   <213> Artificial Sequence
<220>
   <223> Synthetic
<400> 94
   His Ser Cys Arg
   1
<210> 95
   <211> 4
   <212> PRT
   <213> Artificial Sequence
<220>
   <223> Synthetic
<400> 95
   Pro Ser Cys Arg
   1
<210> 96
   <211> 4
   <212> PRT
   <213> Artificial Sequence
<220>
   <223> Synthetic
<400> 96
   His Thr Cys Arg
   1
<210> 97
   <211> 4
   <212> PRT
   <213> Artificial Sequence
<220>
   <223> Synthetic
<400> 97
   Pro Thr Cys Arg
   1
<210> 98
   <211> 4
   <212> PRT
   <213> Artificial Sequence
<220>
   <223> Synthetic
<400> 98
   His Ser Cys Lys
   1
<210> 99
   <211> 4
   <212> PRT
   <213> Artificial Sequence
<220>
   <223> Synthetic
<400> 99
   Pro Ser Cys Lys
   1
<210> 100
   <211> 4
   <212> PRT
   <213> Artificial Sequence
<220>
   <223> Synthetic
<400> 100
   His Thr Cys Lys
   1
<210> 101
   <211> 4
   <212> PRT
   <213> Artificial Sequence
<220>
   <223> Synthetic
<400> 101
   Pro Thr Cys Lys
   1
<210> 102
   <211> 6
   <212> PRT
   <213> Artificial Sequence
<220>
   <223> Synthetic
<400> 102
   Gly Arg Gly Asp Ser Pro
   1 5
<210> 103
   <211> 6
   <212> PRT
   <213> Artificial Sequence
<220>
   <223> Synthetic
<400> 103
   Gly Arg Gly Glu Ser Pro
   1 5
<210> 104
   <211> 5
   <212> PRT
   <213> Artificial Sequence
<220>
   <223> Synthetic
<400> 104
   Gly Arg Gly Asp Ser
   1 5
<210> 105
   <211> 172
   <212> PRT
   <213> Artificial Sequence
<220>
   <223> Synthetic
<400> 105
   Pro Leu Ser Pro Pro Thr Asn Leu His Leu Glu Ala Asn Pro Asp Thr
   1               5                   10                  15
   Gly Val Leu Thr Val Ser Trp Glu Arg Ser Thr Thr Pro Asp Ile Thr
               20                  25                  30
   Gly Tyr Arg Ile Thr Thr Thr Pro Thr Asn Gly Gln Gln Gly Asn Ser
           35                  40                  45
   Leu Glu Glu Val Val His Ala Asp Gln Ser Ser Ala Val Pro Pro Pro
       50                  55                  60
   Thr Asp Leu Arg Phe Thr Asn Ile Gly Pro Asp Thr Met Arg Val Thr
   65                  70                  75                  80
   Trp Ala Pro Pro Pro Ser Ile Asp Leu Thr Asn Phe Leu Val Arg Tyr
                   85                  90                  95
   Ser Pro Val Lys Asn Glu Glu Asp Val Ala Glu Leu Ser Ile Ser Pro
               100                 105                 110
   Ser Asp Asn Ala Gly Leu Asp Ser Pro Thr Gly Ile Asp Phe Ser Asp
           115                 120                 125
   Ile Thr Ala Asn Ser Phe Thr Val His Trp Ile Ala Pro Arg Ala Thr
       130                 135                 140
   Ile Thr Gly Tyr Arg Ile Arg His His Pro Glu His Phe Ser Gly Arg
   145                 150                 155                 160
   Pro Arg Glu Asp Arg Val Pro His Ser Arg Asn Ser
                   165                 170